# EUROPÄISCHE PATENTSCHRIFT

(11) **EP 0 759 429 B1**
(45) Veröffentlichungstag und Bekanntmachung des Hinweises auf die Patenterteilung: **24.10.2001**
(21) Anmeldenummer: 96112856.8
(22) Anmeldetag: 09.08.1996
(51) Int. Cl.: C07D 213/73, C07D 213/77, C07D 213/64, C07D 401/04, A01N 43/56

(54) **Substituierte Trifluormethylpyridine verwendbar als Zwischenprodukte für herbizide 1-(Pyridyl)-Pyrazole**
Substituted trifluormethylpyridines useful as intermediates for herbicidal 1-(pyridyl)-pyrazoles
Trifluorméthylpyridines substituées utiles comme intermédiaires pour 1-(pyridyl)-pyrazoles herbicides

(30) Priorität: 21.08.1995 DE 19530605
(43) Veröffentlichungstag der Anmeldung: 26.02.1997
(73) Patentinhaber: BASF Aktiengesellschaft, 67063 Ludwigshafen (DE)
(72) Erfinder: Hamprecht, Gerhard, Dr., 69469 Weinheim (DE); Heistracher, Elisabeth, Dr., 68159 Mannheim (DE); Klintz, Ralf, Dr., 67269 Grünstadt (DE); Schäfer, Peter, Dr., 67308 Ottersheim (DE); Zagar, Cyrill, Dr., 67061 Ludwigshafen (DE)

(56) Entgegenhaltungen:
- EP-A- 0 031 218
- EP-A- 0 207 285
- US-A- 4 563 530
- US-A- 5 156 656
- US-A- 5 167 691

## Beschreibung

Die Erfindung betrifft neue substituierte Trifluormethylpyridine der allgemeinen Formel I, 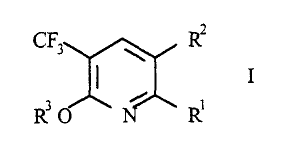 in der die Substituenten die folgende Bedeutung haben:
- R¹: Amino oder Hydrazino;
- R²: Halogen; und
- R³: C₁-C₆ Alkyl, C₂-C₆ Alkenyl oder C₂-C₆-Alkinyl, wobei diese Gruppen bis zu 6 Halogenatome tragen können,
C₃-C₆-Cycloalkyl, das bis zu 3 C₁-C₃-Alkylreste tragen kann, C₁-C₆-Cyanoalkyl, C₁-C₄-Alkoxy-C₂-C₆-alkyl, 3-Oxetanyl, Carboxyl-C₁-C₆-alkyl, C₁-C₆-Alkoxycarbonyl-C₁-C₆-Alkyl, C₁-C₄-Alkoxy-C₂-C₄-alkoxycarbonyl-C₁-C₆-alkyl, Aminocarbonyl-C₁-C₆-alkyl, C₁-C₄-Alkylaminocarbonyl-C₁-C₆-alkyl, C₁-C₄-Dialkylaminocarbonyl-C₁-C₆-alkyl, C₂-C₄-Alkenylaminocarbonyl-C₁-C₆-alkyl, C₃-C₄-Alkinylaminocarbonyl-C₁-C₆-alkyl, C₁-C₄-Alkyl-C₃-C₄-alkenylaminocarbonyl-C₁-C₆-alkyl, C₁-C₄-Alkyl-C₃-C₄-alkinylaminocarbonyl-C₁-C₆-alkyl, C₃-C₆-(α-Alkylalkyliden)iminoxy-C₂-C₆-alkyl, Cyclopropylmethyl C₁-C₆-Alkylamino, C₁-C₆-Dialkylamino, C₁-C₆-Alkylidenimino- oder α-(C₁-C₄-Alkyl)-C₂-C₆-alkylidenimino, und
Halogen Fluor, Chlor, Brom oder Jod.

Weiterhin betrifft die Erfindung ein Verfahren zur Herstellung der Trifluormethylpyridine I durch Umsetzung von 2,3,6-Trihalogen-5-trifluormethyl-pyridinen mit Ammoniak oder Hydrazin und Alkoholen bzw. umgekehrt, zuerst mit Alkoholen und dann mit Ammoniak oder Hydrazin.

2-Amino- bzw. 2-Hydrazino-pyridine sind wertvolle Zwischenprodukte für organische Synthesen, insbesondere für die Herstellung von Pflanzenschutzmitteln, z. B. herbizid wirksamen 1-Pyridylpyrazol-derivaten.

Die US 5,167,691 beschreibt 1-Pyridyl-pyrazole, die eine schlechtere herbizide Wirkung aufweisen als die 1-Pyridyl-pyrazole, die auf den erfindungsgemäßen Trifluormethylpyridinen der allgemeinen Formel I beruhen. In Beispiel 4 wird (3,6-Dichlor-5-trifluoromethylpyridin-2-yl)hydrozin beschrieben, das sich durch die unterschiedlichen Substituenten von den erfindungsgemäßen Trifluormethylpyridinen I unterscheidet.

Die EP-A 0 207 285 beschreibt 5-Amino-1-pyridyl-pyrazole. Alle in der Tabelle 1 aufgeführten Pyridyl-Substituenten sind in der 6-Position nicht substituiert im Unterschied zu den Substituenten der erfindungsgemäßen Trifluormethylpyridine I.

Während 2-Amino-3,6-dichlor-5-trifluormethylpyridin bereits länger bekannt ist (USP 4349681), sind Umsetzungsprodukte, die sich durch nucleophile Reaktion von Alkoholen oder deren Salzen von dem entsprechenden 6-Chlor-pyridinderivat ableiten, bisher nicht beschrieben. In eigenen Versuchen wurde gefunden, daß sich beispielsweise 2-Amino-3,6-dichlor-5-trifluormethylpyridin mit Natriummethylat unter Rückfluß nicht umsetzt und daß in Gegenwart von Kaliumhydroxid bereits bevorzugt Verseifung der 5-CF₃-Gruppe zu dem entsprechenden Carbonsäuremethylester erfolgt. Erst in einem speziellen Lösungsmittelgemisch mit DMF konnten nach 90 Stunden Erhitzen Spuren von 2-Amino-3-chlor-6-methoxy-5-trifluormethylpyridin neben mehreren Verunreinigungen nachgewiesen werden. Die Ausbeute ist mit 5 % jedoch unbefriedigend.

Wird umgekehrt das neue 2,5-Dichlor-6-methoxy-3-trifluormethylpyridin mit Ammoniak umgesetzt, so tritt unter Verlust der Methoxygruppe überwiegend die Bildung des unerwünschten 2-Amino-3,6-dichlor-5-trifluormethylpyridins ein.

Vor diesem Hintergrund ist verständlich, daß 2,6-(bzw. 6,2-) Alkoxy, -amino- (oder -hydrazino-) halogen-trifluormethylpyridine bisher nicht bekannt wurden.

Es wurde nun gefunden, daß man die neuen Trifluormethylpyridine der allgemeinen Formel I 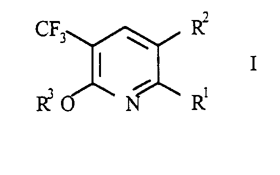 in der die Substituenten die folgende Bedeutung haben:
- R¹: Amino oder Hydrazino;
- R²: Halogen; und
- R³: C₁-C₆-Alkyl, C₂-C₆-Alkenyl oder C₂-C₆-Alkinyl, wobei diese Gruppen bis zu 6 Halogenatome tragen können,
C₃-C₆-Cycloalkyl, das seinerseits bis zu 3 C₁-C₃-Alkylreste tragen kann, C₁-C₆-Cyanoalkyl, C₁-C₄-Alkoxy-C₂-C₆-alkyl, 3-Oxetanyl, Carboxyl-C₁-C₆-alkyl, C₁-C₆-Alkoxycarbonyl-C₁-C₆-alkyl, C₁-C₄-Alkoxy-C₂-C₄-alkoxycarbonyl-C₁-C₆-alkyl, C₁-C₄-Alkylaminocarbonyl-C₁-C₆-alkyl, Aminocarbonyl-C₁-C₆-alkyl, C₁-C₄-Dialkylaminocarbonyl-C₁-C₆-alkyl, C₂-C₄-AlkenylaminocarbonylC₁-C₆-alkyl, C₃-C₄-Alkinylaminocarbonyl-C₁-C₆-alkyl, C₁-C₄-Alkyl-C₃-C₄-alkenylaminocarbonyl-C₁-C₆-alkyl, C₁-C₄-Alkyl-C₃-C₄-alkinylaminocarbonyl-C₁-C₆-alkyl, C₃-C₆-(α-Alkyl-alkyliden)iminoxy-C₂-C₆-alkyl, Cyclopropylmethyl, C₁-C₆-Alkylamino, C₁-C₆-Dialkylamino, C₁-C₆-Alkylidenimino- oder α-(C₁-C₄-Alkyl)-C₂-C₆-alkylidenimino und
Halogen Fluor, Chlor, Brom oder Jod,
vorteilhaft erhält, wenn man 2,3,6-Tichlor-5-trifluormethylpyridin der Formel II 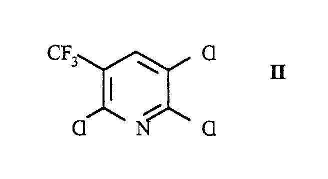
in einem ersten Schritt einem Halogenaustausch zu dem entsprechenden 3-Chlor-2,6-difluor-5-trifluormethylpyridin der Formel III unterzieht 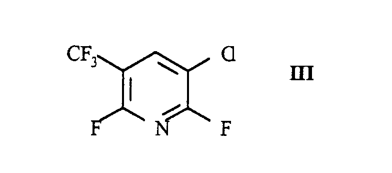
und dieses dann nacheinander mit Ammoniak oder Hydrazin zu dem 2-Amino- bzw. 2-Hydrazino-3-chlor-6-fluor-5-trifluormethylpyridin der Formel IV oder IV' 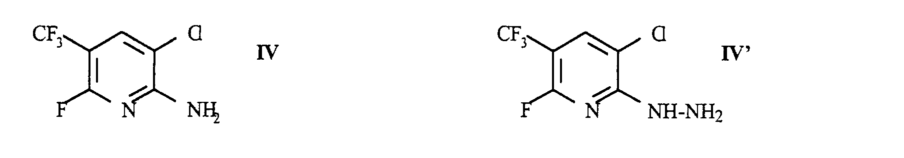
und schließlich mit einem Alkohol der Formel V,

   HO-R³ V

   in der R³ die vorgenannte Bedeutung hat, oder dessen Alkali- oder Erdalkalisalz zu den Verbindungen der Formel I umsetzt.

Die Umsetzung kann im Falle der Darstellung der Verbindungen I unter Verwendung von 2,3,6-Trichlor-5-trifluormethylpyridin und Kaliumfluorid als Halogenaustauschmittel sowie der Nucleophile Ammoniak und Methanol durch folgendes Schema beschrieben werden:

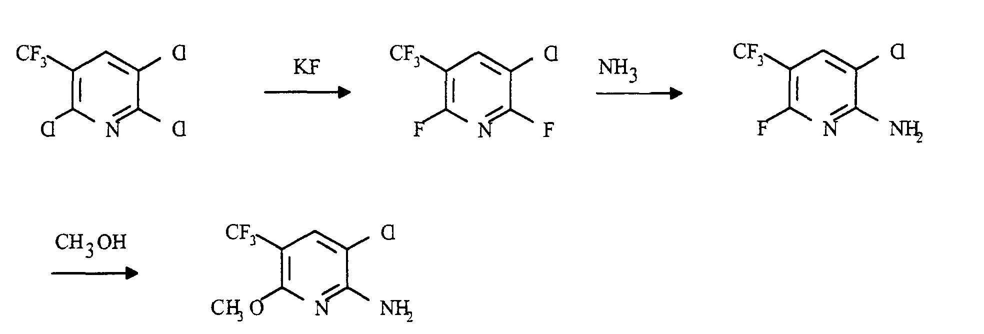

Bei Verwendung von Hydrazin an Stelle von Ammoniak verläuft die Umsetzung analog nach folgendem Schema:

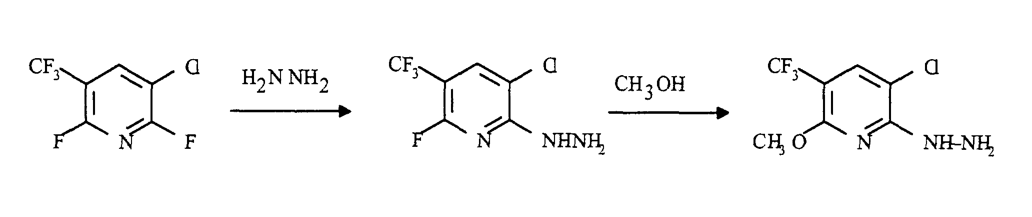

Das Verfahren liefert auf einfachem und wirtschaftlichem Weg neue 2-Amino bzw. 2-Hydrazino-halogen-trifluormethylpyridine in hoher Ausbeute und Reinheit. Entgegen der Erwartung wird die Trifluormethylgruppe nicht verseift noch eine bereits eingeführte Alkoxygruppe durch Ammoniak wieder eliminiert. Im Hinblick auf den Stand der Technik sind alle diese vorteilhaften Eigenschaften überraschend. Die vorstehend für den Substituenten R³ in der Formel I genannten Bedeutungen stellen Sammelbegriffe für individuelle Aufzählungen der einzelnen Gruppenmitglieder dar. Sämtliche Kohlenstoffketten, also alle Alkyl-, Alkenyl-, Alkinyl-, Halogenalkyl-, Halogenalkenyl-, Halogenalkinyl- und Halogenalkoxyteile können geradekettig oder verzweigt sein. Halogenierte Substituenten tragen vorzugsweise 1 - 6 gleiche oder verschiedene Halogenatome.

Im einzelnen bedeuten beispielsweise:
- Halogen: Fluor, Chlor, Brom und Jod, vorzugsweise Fluor und Chlor;
- C₁-C₄-Alkyl: Methyl, Ethyl, n-Propyl, 1-Methylethyl, n-Butyl, 1-Methylpropyl, 2-Methylpropyl und 1,1-Dimethylethyl;
- C₁-C₆-Alkyl: C₁-C₄-Alkyl wie vorstehend genannt, sowie n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-2-methylpropyl;
- C₁-C₈-Alkyl: C₁-C₆-Alkyl wie vorstehend genannt, sowie u. a. n-Heptyl, n-Octyl;
- C₂-C₄-Alkenyl: Ethenyl, Prop-1-en-1-yl, Prop-2-en-1-yl, 1-Methylethenyl, n-Buten-1-yl, N-Buten-2-yl, n-Buten-3-yl, 1-Methyl-prop-1-en-1-yl, 2-Methyl-prop-1-en-1-yl, 1-Methylprop-2-en-1-yl und 2-Methyl-prop-2-en-1-yl;
- C₃-C₆-Alkenyl: Prop-1-en-1-yl, Prop-2-en-1-yl, 1-Methylethenyl, n-Buten-1-yl, n-Buten-2-yl, n-Buten-3-yl, 1-Methylprop-1-en-1-yl, 2-Methyl-prop-1-en-1-yl, 1-Methylprop-2-en-1-yl, 2-Methyl-prop-2-en-1-yl, n-Penten-1-yl, n-Penten-2-yl, n-Penten-3-yl, n-Penten-4-yl, 1-Methylbut-1-en-1-yl, 2-Methyl-but-1-en-1-yl, 3-Methylbut-1-en-1-yl, 1-Methyl-but-2-en-1-yl, 2-Methylbut-2-en-1-yl, 3-Methyl-but-2-en-1-yl, 1-Methylbut-3-en-1-yl, 2-Methyl-but-3-en-1-yl, 3-Methylbut-3-en-1-yl, 1,1-Dimethyl-prop-2-en-1-yl, 1,2-Dimethylprop-1-en-1-yl, 1,2-Dimethyl-prop-2-en-1-yl, 1-Ethylprop-1-en-2-yl, 1-Ethyl-prop-2-en-1-yl, n-Hex-1-en-1-yl, n-Hex-2-en-1-yl, n-Hex-3-en-1-yl , n-Hex-4-en-1-yl, n-Hex-5-en-1-yl, 1-Methyl-pent-1-en-1-yl, 2-Methylpent-1-en-1-yl, 3-Methyl-pent-1-en-1-yl, 4-Methylpent-1-en-1-yl, 1-Methyl-pent-2-en-1-yl, 2-Methylpent-2-en-1-yl, 3-Methyl-pent-2-en-1-yl, 4-Methylpent-2-en-1-yl, 1-Methyl-pent-3-en-1-yl, 2-Methylpent-3-en-1-yl, 3-Methyl-pent-3-en-1-yl, 4-Methylpent-3-en-1-yl, 1-Methyl-pent-4-en-1-yl, 2-Methylpent-4-en-1-yl, 3-Methyl-pent-4-en-1-yl, 4-Methylpent-4-en-1-yl, 1,1-Dimethyl-but-2-en-1-yl, 1,1-Dimethylbut-3-en-1-yl, 1,2-Dimethyl-but-1-en-1-yl, 1,2-Dimethylbut-2-en-1-yl, 1,2-Dimethyl-but-3-en-1-yl, 1,3-Dimethylbut-1-en-1-yl, 1,3-Dimethyl-but-2-en-1-yl, 1,3-Dimethylbut-3-en-1-yl, 2,2-Dimethyl-but-3-en-1-yl, 2,3-Dimethylbut-1-en-1-yl, 2,3-Dimethyl-but-2-en-1-yl, 2,3-Dimethylbut-3-en-1-yl, 3,3-Dimethyl-but-1-en-1-yl, 3,3-Dimethylbut-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethylbut-2-en-1-yl, 2-Ethyl-but-3-en-1-yl, 1,1,2-Trimethylprop-2-en-1-yl, 1-Ethyl-1-methyl-prop-2-en-1-yl, 1-Ethyl-2-methyl-prop-1-en-1-yl und 1-Ethyl-2-methylprop-2-en-1-yl, vorzugsweise Ethenyl und Prop-2-en-1-yl;
- C₂-C₆-Alkinyl: Ethinyl und C₃-C₆-Alkinyl wie Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methylpent-1-in-5-yl, 4-Methyl-pent-1-in-1-yl, 4-Methylpent-2-in-4-yl und 4-Methyl-pent-2-in-5-yl, vorzugsweise Prop-2-in-1-yl, 1-Methyl-prop-2-in-1-yl;
- C₂-C₆-Halogenalkenyl: C₂-C₆-Alkenyl wie vorstehend genannt, wobei jeweils 1 - 6 Wasserstoffatome durch Fluor, Chlor und / oder Brom ersetzt sind;
- C₂-C₆-Halogenalkinyl: C₂-C₆-Alkinyl wie vorstehend genannt, wobei jeweils ein bis sechs Wasserstoffatome durch Fluor, Chlor und / oder Brom ersetzt sind;
- C₃-C₆-Cycloalkyl: Cyclopropyl, Cyclobutyl, Cyclopentyl und Cyclohexyl, vorzugsweise Cyclopropyl, Cyclopentyl und Cyclohexyl;
- C₁-C₆-Halogenalkyl: C₁-C₄-Alkyl wie vorstehend genannt, das partiell oder vollständig durch Fluor, Chlor und / oder Brom substituiert ist, also z. B., Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 3-Chlorpropyl, vorzugsweise Trifluormethyl;
- Cyano-(C₁-C₆)-alkyl: C₁-C₆-Alkyl die vorstehend genannt, wobei jeweils ein Wasserstoffatom durch die Cyanogruppe ersetzt ist, also z. B. Cyanomethyl, 1-Cyanoeth-1-yl, 2-Cyanoeth-1-yl, 1-Cyano-prop-1-yl, 2-Cyano-prop-1-yl, 3-Cyanoprop-1-yl, 1-Cyano-prop-2-yl, 2-Cyano-prop-2-yl, 1-Cyanobut-1-yl, 2-Cyano-but-1-yl, 3-Cyano-but-1-yl, 4-Cyanobut-1-yl, 1-Cyano-but-2-yl, 2-Cyano-but-2-yl, 1-Cyanobut-3-yl, 2-Cyano-but-3-yl, 1-Cyano-2-methyl-prop-3-yl, 2-Cyano-2-methyl-prop-3-yl, 3-Cyano-2-methyl-prop-3-yl, und 2-Cyano-methyl-prop-2-yl, vorzugsweise Cyanomethyl, 1-Cyano-1-methylethyl;
- C₁-C₄-Alkoxy: Methoxy, Ethoxy, n-Propoxy, 1-Methylethoxy, n-Butoxy, 1-Methyl-propoxy, 2-Methylpropoxy und 1,1-Dimethylethoxy, vorzugsweise Methoxy, Ethoxy und 1-Methylethoxy;
- C₁-C₄-Alkylamino: Methylamino, Ethylamino, n-Propylamino,1-Methylethylamino, n-Butylamino, 1-Methylpropylamino, 2-Methylpropylamino und 1,1-Dimethylethylamino, vorzugsweise Methylamino und Ethylamino;
- Di-(C₁-C₄-alkyl)amino: N,N-Dimethylamino, N,N-Diethylamino, N,N-Dipropylamino, N,N-Di-(1-methylethyl)amino, N,-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N- (2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methyl-propyl)amino, N-Ethyl-N-(2-methyl-propyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-methylethyl)-N-(1-methylpropyl)amino, N-(1-Methylethyl)-N-(2-methyl-propyl)amino, N-(1,1-Dimethylethyl)-N-(1-methylethyl)amino, N-Butyl-N-(1-methylpropyl)amino, N-Butyl-N-(2-methyl-propyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino, vorzugsweise Dimethylamino und Diethylamino;
   C₁-C₄-Alkylaminocarbonyl: Methylaminocarbonyl, Ethylaminocarbonyl, n-Propylaminocarbonyl, 1-Methylethylaminocarbonyl, n-Butylaminocarbonyl, 1-Methylpropylaminocarbonyl, 2-Methylpropylaminocarbonyl und 1,1-Dimethylethylaminocarbonyl, vorzugsweise Methylaminocarbonyl und Ethylaminocarbonyl;
- Di-(C₁-C₄-alkyl)aminocarbonyl: N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl)aminocarbonyol, N,N-Di-(2-methylpropyl)aminocarbonyl, N,N-Di-(1,1-dimethylethyl)aminocarbonyl, N-Ethyl-N-methylaminocarbonyl, N-methyl-N-propylaminocarbonyl, N-Methyl-N-(1-methylethyl)aminocarbonyl, N-Butyl-N-methylaminocarbonyl, N-Methyl-N-(1-methylpropyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-methylpropyl)-N-propylaminocarbonyl, N-(1,1-Dimethylethyl)-N-propylaminocarbonyl, N-Butyl-N-(1-methyl-ethyl)aminocarbonyl, N-(1-Methylethyl)-N-(1-methylpropyl)aminocarbonyl, N-(1-Methylethyl)-N-(2-methylpropyl)aminocarbonyl, N-(1,1-Dimethylethyl)-N-(1-methylethyl)aminocarbonyl, N-Butyl-N-(1-methylpropyl)aminocarbonyl, N-Butyl-N-(2-methylpropyl)aminocarbonyl, N-Butyl-N-(1,1-dimethylethyl)aminocarbonyl, N-(1-Methylpropyl)-N-(2-methyl-propyl)aminocarbonyl, N-(1,1-Dimethylethyl)-N-(1-methylpropyl)aminocarbonyl und N-(1,1-Dimethylethyl)-N-(2-methylpropyl)aminocarbonyl, vorzugsweise Dimethylaminocarbonyl und Diethylaminocarbonyl;
- C₁-C₆-Alkylidenimino; Methylidenimino, Ethylidenimino, Propylidenimino, Butylidenimino, Pentylidenimio, Hexylidenimino, vorzugsweise Methylidenimino und Ethylidenimino;
- α-(C₁-C₂-Alkyl)-C₂-C₄-alkylidenimino: α-Methylethylidenimino, α-Methyl-propylidenimino, α-Ethyl-ethylidenimino, α-Ethylpropylidenimino, α-Methyl-butylidenimino, vorzugsweise α-Methylethylidenimino und α-Methyl-propylidenimino.

Ferner sind unter den Verbindungen I diejenigen besonders bevorzugt, in denen
- R¹: Amino oder Hydrazino;
- R²: Chlor oder Fluor;
- R³: C₁-C₄-Alkyl, C₂-C₄-Alkenyl, oder C₂-C₄-Alkinyl, wobei diese Gruppen bis zu 6 Halogenatome tragen können, C₃-C₆-Cycloalkyl, das seinerseits bis zu 3 Methylrest tragen kann, C₁-C₂-Alkoxy-C₂-C₄-alkoxycarbonyl-C₁-C₄-alkyl, C₁-C₄-Cyanoalkyl, C₁-C₂-Alkoxy-C₂-C₄-alkyl, 3-Oxetanyl, Carboxyl-C₁-C₄-alkyl, C₁-C₄-Alkoxycarbonyl-C₁-C₄-alkyl, C₁-C₂-Alkoxy-C₂-C₄-alkoxycarbonyl-C₁-C₄-alkyl, C₁-C₂-Alkaminocarbonyl-C₁-C₄-alkyl, C₃-C₄- (α-Alkyliden)iminoxy-C₂-C₄-alkl, Cyclopropylmethyl, C₁-C₄-Alkylamino, C₁-C₄-Dialkylamino, C₁-C₄-Alkylidenimino oder α-(C₁-C₂-Alkyl)-C₂-C₄-Alkylidenimino bedeuten.

Das für die Herstellung der Verbindungen IV erforderliche Ausgangsmaterial III - 3-Chlor-2,6-difluor-5-trifluormethylpyridin - wird in US-A 5 156 656 verwendet; seine Herstellung ist in der Literatur bisher jedoch noch nicht beschrieben.

Die neuen substituierten Pyridine I sind auf verschiedene Weise erhältlich, vorzugsweise nach einem der folgenden Verfahren:

Der Halogenaustausch des 2,3,6-Trichlor-5-trifluormethylpyridins II wird bevorzugt in Gegenwart eines polaren Lösungsmittels mit Kaliumfluorid bei Temperaturen zwischen 100 - 180°C vorzugsweise 130 - 170°C durchgeführt.

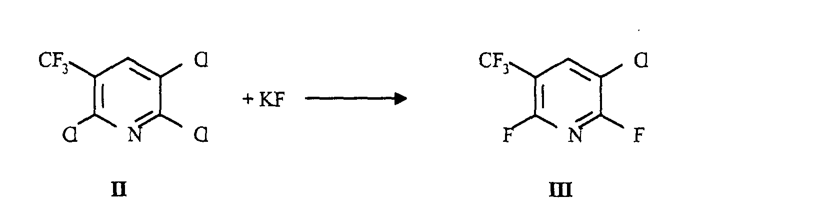

Als Lösungsmittel verwendet man für diese Umsetzungen Nitrile wie Acetonitril, Propionitril, Butyronitril, Isobutyronitril, Glykolether wie Dimethylglykolether. Diethylglykolether, Diethylenglykoldimethylether, Carbonsäureamide wie DMF, N-Methylpyrrolidon, Harnstoffe wie Tetraethylharnstoff, Tetrabutylharnstoff, Dimethylethylenharnstoff, Dimethylpropylenharnstoff, Sulfoxide wie Dimethylsulfoxid und vorzugsweise Sulfone wie Dimethylsulfon, Diethylsulfon, Tetramethylensulfon (Sulfolan) oder Pentamethylensulfon. Auch die Durchführung in der Schmelze ohne Zusatz eines Lösungsmittels ist erfindungsgemäß möglich.

Der Halogenaustausch verläuft bereits ohne Katalysator mit hoher Geschwindigkeit. Er kann jedoch durch Zugabe eines Katalysators, z. B. eines Kronenethers oder Cryptanden beschleunigt werden. Diese sind organische Komplexliganden, die insbesondere gut zur Bindung von Alkali dienen. Die Cryptanden liefern eine dreidimensionale Umhüllung. Bezüglich Herstellung dieser Stoffe, siehe "Kontakte" (1977), Seiten 11 - 31 und 36 - 48. Als Katalysator sind Kronenether bevorzugt, von denen z. B. die folgenden Verbindungen genannt sein sollen: 12-Krone-4, 14-Krone-4, Dibenzo-14-krone-4, 18-Krone-5, 18-Krone-6, Dibenzo-18-krone-6 oder Aza-18-krone-6.

Diese Katalysatoren werden zweckmäßig in einer Menge von 0,05 - 5, insbesondere 0,1 - 2 Molprozent je Mol Ausgangsstoff II eingesetzt.

Die molaren Verhältnisse, in denen die Ausgangsverbindungen miteinander umgesetzt werden, betragen 1,9 - 2,8, vorzugsweise 2 - 2,4 für das Verhältnis von Kaliumfluorid zu Pyridinderivat II. Die Konzentration der Edukte im Lösungsmittel beträgt 0,1 - 5 mol/l, bevorzugt 0,2 - 2 mol/l.

Besonders vorteilhaft lassen sich die Verbindungen III herstellen, wenn man vor dem eigentlichen Halogenaustausch das Pyridinderivat II beispielsweise in Gegenwart eines aliphatischen Sulfons bei Temperaturen bis 150°C, zweckmäßig zwischen 50°C und 120°C, insbesondere 70 - 100°C mit 0,1 - 0,4, zweckmäßig 0,15 - 0,3 mol eines Säurechlorids der schwefligen Säure oder Kohlensäure behandelt und das Reaktionsgemisch dann bei Temperaturen von 70 - 250°C, vorzugsweise 80 - 200°C mit Kaliumfluorid umsetzt.

Als Katalysatoren sind für diese Verfahrensstufe z. B. N,N-disubstituierte Carbonamide wie DMF, N,N-Dimethylacetamid oder N,N-Diisopropylacetamid geeignet. Der Katalysator wird zweckmäßig in einer Menge von 0,2 - 2 Gewichtsprozent bezogen auf das Säurechlorid eingesetzt.

Bei der Umsetzung mit dem Säurechlorid erhitzt man zweckmäßig so lange, bis keine Gasentwicklung mehr erfolgt. Es empfiehlt sich, überschüssiges Säurechlorid z. B. durch Einblasen eines Inertgases, wie Stickstoff, oder durch Anlegen von Vakuum zu entfernen.

Zu diesem Gemisch gibt man dann das Kaliumfluorid, das zweckmäßig vorher getrocknet wurde und hält die durch Verrühren erhaltene Mischung 1 - 10 Stunden auf Reaktionstemperatur.

Als Fluoridsalze kommen erfindungsgemäß neben Kaliumfluorid auch Tetraalkyl-(C₁-C₁₃)-ammoniumfluorid sowie entsprechende Mischungen untereinander oder mit Cäsiumfluorid oder Rubidiumfluorid in Frage, wobei diese Mischungen mit Cäsiumfluorid nicht mehr als 50 Gew.-% an Cäsiumfluorid enthalten. Bevorzugt werden Fluoridmischungen verwendet, die zumindest 75 Gew.-% Kaliumfluorid enthalten; insbesondere bestehen derartige Mischungen aus wenigstens 90 Gew.-% Kaliumfluorid und höchstens 10 Gew.-% Cäsiumfluorid oder aus 60 Gew.-% Kaliumfluorid und 40 Gew.-% Rubidiumfluorid. In einer weiteren bevorzugten Ausgestaltungsform kommt als Fluoridsalz nur Kaliumfluorid zum Einsatz.

Als Phasentransferkatalysatoren können quartäre Ammonium- oder Phosphoniumsalze verwendet werden. An geeigneten Verbindungen seien folgende genannte: Tetraalkyl-(C₁-C₁₈)-ammoniumchloride, -bromide oder -fluoride, Tetraalkyl-(C₁-C₁₈)-phosphoniumchloride oder -bromide, Tetraphenylphosphoniumchlorid oder -bromid, (Phenyl)ₘ (alkyl-(C₁-C₁₈))ₙ-phosphoniumchloride oder -bromide, wobei m = 1 - 3, n = 3 - 1 und m + n = 4 ist. Auch Gemische dieser Salze sind einsetzbar. Bei geeigneter Wahl des Katalysators für die jeweilige umzusetzende Verbindung, was durch einige Routineversuche leicht zu ermitteln ist, erhält man hohe Raumleistungen und Ausbeuten; außerdem sind die Apparatetotzeiten sowie die gesamten Apparatekosten nach dem erfindungsgemäßen Verfahren besonders günstig.

Die Menge an Phasentransferkatalysator beträgt im allgemeinen bis zu 20 Gew.-%, bevorzugt zwischen 3 und 15 Gew.-% und besonders bevorzugt zwischen 3 - 8 Gew.-%, bezogen auf die eingesetzte Menge an Fluoridsalz.

Als Phasentransferkatalysatoren können auch Oligo- oder Polyalkylenglykoldimethylether verwendet werden, wobei der Alkylenrest 2 - 6 C-Atome, vorzugsweise 2 und / oder 3-C-Atome enthält, also vorzugsweise den Ethylen- und / oder den Propylenrest und insbesondere nur den Ethylenrest darstellt. Die Zahl der O-Ethylen(glykol)einheiten (-O-CH₂-CH₂-)ₙ und / oder der O-Propyleneinheiten in diesen Verbindungen kann von n = 4 (z. B. Tetraethylenglykoldimethylether) bis etwa n = 150 betragen; bevorzugt werden jedoch Ether eingesetzt, deren Polymerisationsgrad zwischen n = 4 und n = 25 beträgt. Im Falle von Alkylenresten mit mehr als 3 C-Atomen liegt n im allgemeinen nicht höher als 6. Die Einsatzmenge dieser Ether, insbesondere Glykolether liegt zumeist zwischen etwa 0,6 Gew.-% und etwa 200 Gew.-%, bevorzugt zwischen etwa 5 und etwa 100 Gew.-% und besonders bevorzugt zwischen etwa 10 und etwa 50 Gew.-%, bezogen auf die Menge des eingesetzten Fluoridsalzes. Der besondere Vorteil bei der Verwendung dieser Verbindungen liegt darin, daß meist der Einsatzmenge entsprechend weniger Lösungsmittel verwendet werden kann, da die Glykolether bei der Reaktionstemperatur im allgemeinen flüssig werden. Auch Gemische dieser Ether untereinander als auch Gemische dieser Ether (einzeln oder im Gemische) mit den quartären Ammonium- oder Phosphoniumsalzen, vorzugsweise Glykolether mit quartären Phosphoniumsalzen, können eingesetzt werden.

Verwendet man als Fluoridsalz Tetraalkyl-(C₁-C₁₈)-ammoniumfluoride, so ist der Zusatz eines weiteren Phasentransferkatalysators nicht erforderlich, da das Fluoridsalz selbst einen solchen darstellt, der damit also in stöchiometrischen und größeren Mengen eingesetzt werden kann.

Die Verwendung von sprühgetrocknetem Alkalimetallfluorid im erfindungsgemäßen Verfahren verkürzt dabei zwar zum Teil die Reaktionszeiten, ist aber nicht unbedingt notwendig. Ebenso kann unter Zusatz von säurebindenden Mitteln, wie Alkali- und Erdalkalimetallcarbonaten oder basisch wirkenden Oxiden, wie zum Beispiel Magnesiumoxid, oder entsprechenden Gemischen, gearbeitet werden. Besonders bevorzugt ist hierbei Kaliumcarbonat, das in Anteilen von etwa 1 - etwa 10 Gew.-%, bevorzugt von etwa 4 - etwa 6 Gew.-%, bezogen auf die Fluoridsalz-Menge, verwendet wird.

Die säurebindende Mittel sind für den Reaktionsverlauf im allgemeinen nicht wesentlich. In einigen Fällen wird die Reaktionsgeschwindigkeit durch die Bildung von Fluorwasserstoff während der Reaktion erheblich verringert. In diesen Fällen ist es günstig, besonders auch zur Vermeidung von Apparatekorrosion, unter Anwesenheit von derartigen Säurefängern zu arbeiten. Der Einsatz dieser Verbindungen bei Fraktionierung des Reaktionsgemisches oder des Rohproduktes kann aus Gründen der Korrosion in der Fraktionieranlage wünschenswert sein, wobei Magnesiumoxid hierbei besonders bevorzugt ist. Zu diesem Zweck werden der Fraktionierblase bis etwa 10 Gew.-% an Säurefänger zugesetzt, bevorzugt zwischen etwa 3 und 8 Gew.-%, bezogen auf die Gesamtmenge an eingesetzten Destillationssumpf.

Nach der Umsetzung mit Alkalifluorid arbeitet man auf an sich übliche Weise, z. B. durch Filtration, Waschen des Festgutes, Destillation von Filtrat und Waschfiltraten, auf. Im Falle von wassermischbaren Lösungsmitteln kann man die Pyridinderivate Ia auch durch Zugabe von Wasser ausfällen und wie beschrieben aufarbeiten.

Die Umsetzung des 2,6-Difluorpyridins III und der 2-Fluorpyridine VI mit Ammoniak oder Hydrazin bzw. der 2,5-Dichlorpyridine VII mit Hydrazin kann in Abwesenheit oder vorteilhaft in Gegenwart eines Lösungsmittels vorgenommen werden. Als Lösungsmittel sind insbesondere die nachfolgend aufgeführten geeignet:

Kohlenwasserstoffe, z. B. Pentan, Hexan, Heptan, Cyclohexan, Alkohole, z. B. Methanol, Ethanol, n-Propanol, Isopropanol, Butanol, Isobutanol, Ether wie Methyl-tert.-butylether, Diethylether, Ethylpropylether, n-Butylethylether, Di-n-Butylether, Diisobutylether, Diisoamylether, Di-isopropylether, Cyclohexylmethylether, Tetrahydrofuran, 1,2-Dimethoxyethan, Diethylenglykoldimethylether und Anisol, Ester wie Ethylacetat, n-Butylacetat und Isobutylacetat, chlorierte Kohenwasserstoffe wie Methylenchlorid, 1,1,2,2,-Tetrachlorethan, 1,1-Dichlorethylen, 1,2-Dichlorethan, Chlorbenzol, 1,2-, 1,3-, 1,4-Dichlorbenzol, 1-Chlornaphthalin und 1,2,4-Trichlorbenzol, Nitrokohlenwasserstoffe wie Nitromethan, Nitroethan, Nitropropan und Nitrobenzol, dipolare aprotische Lösungsmittel, z. B. Acetonitril, Propionitril, Dimethylformamid, Dimethylacetamid, Dimethylsulfoxid, N-Methylpyrrolidon, 1,3-Dimethyltetrahydro-2-(1H)-pyrimidinon und 1,3-Dimethyl-imidazolidin-2-on, Aromaten, z. B. Benzol, Toluol und Xylol oder Heteroaromaten, z. B. Pyridin, α,β,γ-Picolin und Chinolin, oder Wasser und Gemische dieser Lösungsmittel.

Zweckmäßig verwendet man das Lösungsmittel in einer Menge von 100 - 2000 Gew.-% vorzugsweise 400 - 1200 Gew.-%, bezogen auf die Ausgangsstoffe III, VI oder VII.

Vorteilhaft gibt man 0,9 - 10, insbesondere 1,1 - 5 Molequivalent Ammoniak oder Hydrazinhydrat, bezogen auf die Ausgangsstoffe III, VI oder VII, innerhalb 0,25 - 2 Stunden zu einer Mischung der Ausgangstoffe III, VI oder VII in einem der vorgenannten Lösungsmittel bei 0 - 180°C, vorzugsweise 10 - 130°C und rührt bis zur Vervollständigung der Reaktion (ca. 2 - 20 Stunden) nach.

Setzt man nur ungefähr stöchiometrische Mengen Ammoniak oder Hydrazin ein, so verwendet man zweckmäßig zusätzlich eine organische Hilfsbase, um den entstehenden Halogenwasserstoff abzufangen. Als Hilfbase eignen sich dazu übliche organische Basen wie Trimethylamin, Triethylamin, N-Ethyl-diisopropylamin, Triisopropylamin, N,N-Dimethylanilin, N,N-Dimethylcyclohexylamin, N-Methylpyrrolidin, Pyridin, Chinolin, α,β,γ-Picolin, 2,4- und 2,6-Lutidin und Triethylendiamin. Als Hilfsbase kann man jedoch auch anorganische basische Stoffe z. B. ein Alkali- oder Erdalkalihydroxid wie Lithium-, Natrium-, Kalium-, Calcium-, Magnesium-, oder Zinkhydroxid oder ein Alkali- oder Erdalkalihydrogencarbonat oder -Carbonat der gleichen oben genannten Kationen verwenden. Im allgemeinen sind Zusätze von 0,9 - 1,1 Equivalenten der Hilfsbase, bezogen auf die Ausgangsstoffe III, VI oder VII ausreichend.

Die Reaktion kann drucklos oder unter Druck, kontinuierlich oder diskontinuierlich durchgeführt werden.

Die Aufarbeitung kann in üblicher Weise erfolgen, z. B. extrahiert man das Umsetzungsgemisch mit Wasser zur Entfernung der Salze, trocknet und reinigt die organische Phase, z. B. durch Chromatographie oder Destillation. Man kann jedoch auch direkt die organische Phase einengen und den Rückstand mit einem Lösungsmittel verrühren.

Die Umsetzung der 2,6-Dichlorpyridine II, der 2,6-Difluorpyridine III und der 2-Amino- bzw. 2-Hydrazino-pyridine IV mit einem Alkohol oder dessen Salz kann in Abwesenheit oder vorteilhaft in Gegenwart eines Lösungsmittels vorgenommen werden. Als Lösungsmittel können die vorgenannten eingesetzt werden, ferner Ketone, z. B. Aceton, Methylethylketon oder entsprechende Gemische; man kann jedoch auch den verwendeten Alkohol direkt als Lösungsmittel verwenden.

Zweckmäßig verwendet man das Lösungsmittel in einer Menge von 100 - 2000 Gew.-%, vorzugsweise 400 - 1200 Gew.-%, bezogen auf die Ausgangsstoffe II, III oder IV.

Zur Bindung des bei der Reaktion abgespaltenen Halogenwasserstoffs setzt man zweckmäßig ein Alkali- oder Erdalkalihydroxid wie Lithium- Natrium-,Kalium-, Calcium-, Magnesium- oder Zinkhydroxid, ein Alkali oder Erdalkalihydrogencarbonat oder -carbonat der gleichen oben genannten Kationen oder ein Metallalkoholat, beispielsweise ein Lithium, Natrium oder Kaliumalkoholat zu. Die Alkoholate stellt man zweckmäßig in situ durch Auflösen obengenannter Metalle in dem einzusetzenden Alkohol oder durch Einwirkung von Lithium-, Natrium-, Kalium-, oder Calciumhydrid dar. Man kann jedoch auch eine der vorgenannten organischen Hilsfbasen verwenden.

Vorteilhaft gibt man 0,8 - 1,5, insbesondere 0,9 - 1,2 Molequivalent des Alkohols zweckmäßig in Gegenwart einer equivalenten Menge (einer Base), oder des entsprechenden Alkoholats innerhalb 0,25 - 0,5 Stunden zu einer Mischung der Ausgangstoffe II, III oder IV in einem der vorgenannten Lösungsmittel bei -20 - 100°C, vorzugsweise 0 - 30°C und rührt bis zur Vervollständigung der Reaktion noch 1 - 12 Stunden bei 10 - 120°C vorzugsweise 2 - 10 Stunden bei 20 - 80°C nach und arbeitet dann auf. Man kann jedoch auch umgekehrt den Alkohol zusammen mit einer Base bzw. dem entsprechenden Alkoholat in dem Lösungsmittel vorgeben und dann die Ausgangsstoffe II, III oder IV unter den obigen Bedingungen zugeben.

Die Reaktion kann drucklos oder unter Druck, kontinuierlich oder diskontinuierlich durchgeführt werden.

Die Aufarbeitung kann in gleicher Weise wie für die Umsetzung der Ausgangsstoffe III, VI oder VII beschrieben, durchgeführt werden. Die erfindungsgemäßen Verbindungen sind wertvolle Vorprodukte für die Herstellung von Pflanzenschutzmitteln, insbesondere Herbiziden aus der Klasse der 1-(Pyridyl)-pyrazole oder folgender allgemeinen Formel VIII: 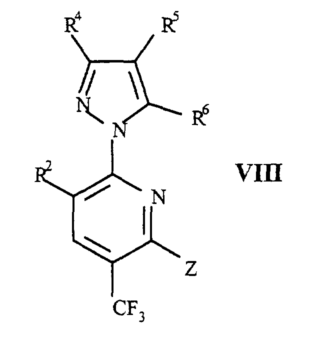 in der die Substituenten die folgende Bedeutung haben:
- Z: OR³; Fluor;
- R²: Halogen;
- R³: C₁-C₆ Alkyl, C₂-C₆ Alkenyl oder C₂-C₆-Alkinyl, wobei diese Gruppen bis zu 6 Halogenatome tragen können,
C₃-C₆-Cycloalkyl, das bis zu 3 C₁-C₃-Alkylreste tragen kann, C₁-C₆-Cyanoalkyl, C₁-C₄-Alkoxy-C₂-C₆-alkyl, 3-Oxetanyl, Carboxyl-C₁-C₆-alkyl, C₁-C₆-Alkoxycarbonyl-C₁-C₆-Alkyl, C₁-C₄-Alkoxy-C₂-C₄-alkoxycarbonyl-C₁-C₆-alkyl, Aminocarbonyl-C₁-C₆-alkyl, C₁-C₄-Alkylaminocarbonyl-C₁-C₆-alkyl, C₁-C₄-Dialkylaminocarbonyl-C₁-C₆-alkyl, C₂-C₄-Alkenylaminocarbonyl-C₁-C₆-alkyl, C₃-C₄-Alkinylaminocarbonyl-C₁-C₆-alkyl, C₁-C₄-Alkyl-C₃-C₄-alkenylaminocarbonyl-C₁-C₆-alkyl, C₁-C₄-Alkyl-C₃-C₄-alkinylaminocarbonyl-C₁-C₆-alkyl, C₃-C₆-(α-Alkylalkyliden)iminoxy-C₂-C₆-alkyl, Cyclopropylmethyl C₁-C₆-Alkylamino, C₁-C₆-Dialkylamino, C₁-C₆-Alkylidenimino- oder α-(C₁-C₄-Alkyl)-C₂-C₆-alkylidenimino,
- R⁴: Wasserstoff, C₁-C₃-Alkyl, Halogen, C₁-C₃-Halogenalkyl;
- R⁵: C₁-C₄-Alkyl, C₂-C₄-Alkenyl, C₂-C₄-Alkinyl, C₁-C₄-Alkoxy, C₃-C₄-Alkenyloxy, C₃-C₄-Alkinyloxy, die durch 1 - 6 Halogenatome substituiert sein können, NO₂, Cyano, Halogen, Rhodano, Amino, ferner einen Rest C(=O)R⁷, S(O)ₙR⁸ oder NH-C(=O)R⁹;
- R⁶: Amino, Halogen, Rhodano, Cyano, Nitro, Hydroxy, C₁-C₃-Alkyl, C₁-C₃-Halogenalkyl, ferner einen Rest S(O)ₙR⁸, N(R¹⁰)R¹¹, OR¹², SR¹³, N=C(R¹⁴)-N(R¹⁵)R¹⁶ oder im Falle von R⁴ = Wasserstoff zusätzlich N=C(R¹⁷)R¹⁸;
- R⁷: Wasserstoff, C₁-C₄-Alkyl, C₂-C₄-Alkenyl, C₂-C₄-Alkinyl, C₁-C₄ Halogenalkyl, C₁-C₄-Alkoxy-C₂-C₄-alkyl, C₃-C₆-Cycloalkyl, C₁-C₄-Alkoxy, C₁-C₄-Alkylamino oder C₁-C₄-Dialkylamino;
- R⁸: C₁-C₄-Alkyl, C₁-C₄-Halogenalkyl, C₁-C₄-Alkoxy, Chlor, Amino oder C₁-C₄-Alkylamino;
- R⁹: C₁-C₄-Alkyl, C₁-C₄-Halogenalkyl, C₁-C₄-Alkoxy-C₁-C₄-alkyl, C₂-C₄-Alkenyl, C₂-C₄-Alkinyl oder C₁-C₄-Alkoxy;
- R¹⁰: Wasserstoff, C₁-C₄-Alkyl oder einen Rest C(=O)R⁷;
- R¹¹: Wasserstoff, C₁-C₄-Alkyl, C₃-C₄-Alkenyl, C₃-C₄-Alkinyl, einen Rest C(=O)R⁷ oder S(O)ₙR⁸;
- R¹²: C₁-C₃-Alkoxy, C₁-C₃-Halogenalkoxy, C₂-C₄-Alkenyloxy, C₃-C₄-Alkinyloxy, C₁-C₅-Alkoxycarbonyl-C₁-C₄-alkyl oder C₁-C₄-Alkoxy-C₂-C₄-alkoxycarbonyl-C₁-C₆-alkyl;
- R¹³: C₁-C₄-Alkoxycarbonyl-C₁-C₄-alkyl oder C₁-C₄-Alkoxy-C₂-C₄-alkoxycarbonyl-C₁-C₆-alkyl;
- R¹⁴: Wasserstoff oder C₁-C₃-Alkyl;
- R¹⁵: C₁-C₄-Alkyl;
- R¹⁶, R¹⁷: unabhängig voneinander Wasserstoff oder C₁-C₄-Alkyl;
- R¹⁸: Wasserstoff, C₁-C₄-Alkyl, C₁-C₄-Halogenalkyl, C₁-C₃-Alkoxy-C₁-C₃-alkyl, C₃-C₆-Cycloalkyl, C₂-C₄-Alkenyl oder C₂-C₄-Alkinyl und
Halogen Fluor, Chlor, Brom oder Jod und
- n: 0, 1 oder 2,
sowie die N-Oxide und die landwirtschaftlich brauchbaren Salze der Verbindungen VIII.

Die 1-(Pyridyl)-pyrazole der Formel VIII sind auf verschiedene Weise erhältlich, beispielsweise nach folgendem Verfahren:

### Verfahren A:

Umsetzung eines Pyridyl-2-hydrazins der Formel IX, in der R² und Z die vorgenannte Bedeutung haben, mit einem Acrylnitrilderivat der Formel X gemäß DBP 3520 330:

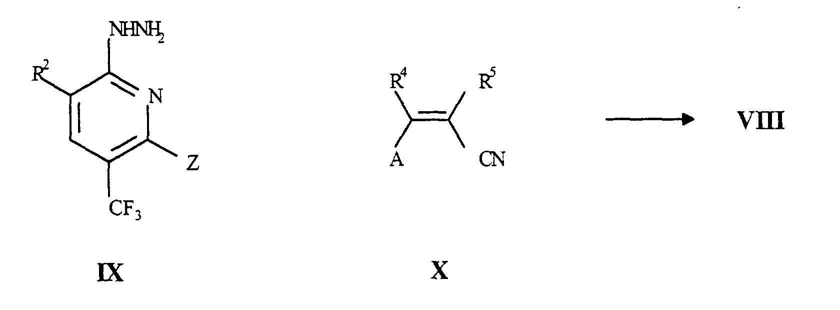

Hierbei haben R⁴ und R⁵ die oben angegebene Bedeutung und A steht für Halogen, Hydroxy, Alkoxy oder Dialkylamino.

Üblicherweise arbeitet man in einem inerten Lösungs- oder Verdünnungsmittel, insbesondere in einem halogenierten Kohlenwasserstoff wie Dichlormethan, 1,2-Dichlormethan, einem Alkohol wie Methanol, Ethanol, n-Propanol, Isopropanol, n-Butanol, Glykol, Methoxyethanol, Ethoxyethanol, Methoxypropanol oder einem Ether wie Tetrahydrofuran, Methyl-tert.-butylether, 1,2-Dimethoxyethan oder 1,2-Diethoxyethan. Die Reaktionstemperatur liegt normalerweise bei 0 180°C, bevorzugt 60 - 140°C.

Üblicherweise werden die Komponenten in etwa stöchiometrischen Mengen eingesetzt, jedoch kann ein Überschuß einer der Komponenten, z. B. im Hinblick auf eine möglichst vollständige Umsetzung der anderen Komponente, vorteilhaft sein.

### Verfahren B:

Hydrolyse und Decarboxylierung eines 1-(Pyridyl-2)-pyrazol-4-carbonesters der Formel VIIIc, in der Z und R² bis R⁴ die vorgenannte Bedeutung haben, gemäß DBP 3520 330.

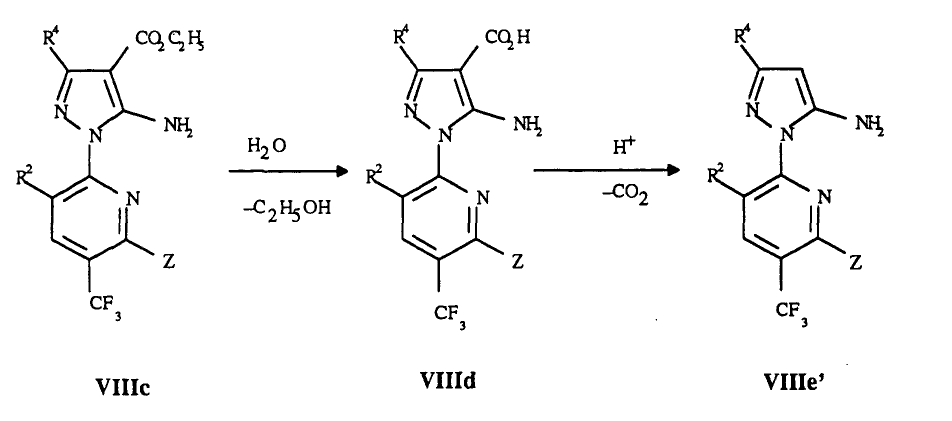

Hierzu kann man einen l-(Pyridyl-2)-pyrazol-4-carbonester mit verdünnter wäßriger Alkalilauge ggf. in Gegenwart wäßrigen Alkohols als Lösungsvermittler bei 40 - 100°C, vorteilhaft 70 - 90°C während 1 - 6 Stunden hydrolisieren und dann durch Ansäuren die Carbonsäure VIIId isolieren. Durch Behandlung mit verdünnter Halogenwasserstoffsäure, zweckmäßig in Gegenwart eines niederen Alkohols als Lösungsvermittler bei 60 - 120°C, vorteilhaft 70 - 90°C erhält man dann das Pyrazolderivat VIIIe'.

Man kann jedoch auch direkt den Pyrazolester der Formel VIIIc mit wäßriger Bromwasserstoffsäure bei 60 - 120°C, vorteilhaft 70 - 90°C behandeln, bis die Verseifung und Decarboxylierung zu VIIIe' beendet ist.

Als Alkalilauge ist Natron- und Kalilauge geeignet.

Als Alkohole kommen Methanol, Ethanol, n-Propanol, Isopropanol oder n-Butanol in Betracht.

Als Halogenwasserstoffsäure sind Chlor-, Brom-, oder Jodwasserstoffsäure geeignet.

### Verfahren C:

Umsetzung eines 1-(Pyridyl-2)-5-aminopyrazols VIIIf mit einer reaktiven Schwefelhalogenverbindung XI oder einer reaktiven Kohlensäureverbindung XII oder einem reaktiven Amidacetal XIII 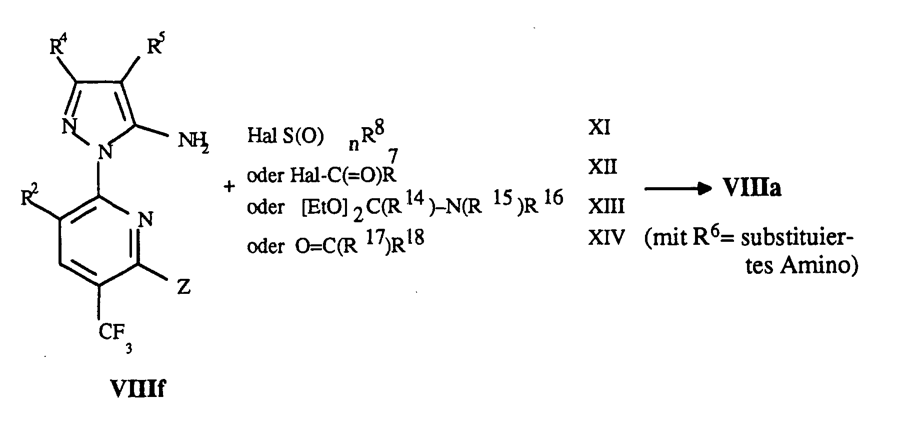 oder einer reaktiven Ketoverbindung XIV gemäß DBP 3520330:

Üblicherweise arbeitet man in einem inerten Lösungs- oder Verdünnungsmittel, insbesondere einem halogenierten Kohlenwasserstoff wie Dichlormethan, 1,2-Dichlorethan, einem Ether wie Diethylether, Methyl-tert.-butylether, Tetrahydrofuran, 1,4-Dioxan oder Anisol, einem Nitril wie Acetonitril, Propionitril oder Butyronitril, einem Ester wie Essigester, Propionsäuremethyl- oder Ethylester oder, falls die Anwesenheit von Wasser nicht stört, ggf. auch in einem Zweiphasengemisch mit Wasser.

Zur Bindung des, bei der Reaktion mit den Ausgangsstoffen XI - XII freiwerdenden Halogenwasserstoffs setzt man zweckmäßig Basen wie beispielsweise Alkalimetallcarbonate und -hydrogencarbonate wie Natriumhydrogencarbonat, Kaliumhydrogencarbonat, Natriumcarbonat und Kaliumcarbonat, Alkalimetallalkoholate wie Natriummethanolat und Kalium-tert.-butanolat, Alkalimetallhydroxide wie Natriumhydroxid oder Kaliumhydroxid ein. Vorteilhaft kommen auch organische Basen in Frage: Trimethylamin, Triethylamin, Pyridin, α-, β-, γ-Picolin, Lutidin, N-Dimethylanilin, N-Diäthylanilin, N-Propyl-piperidin, Chinolin, Isochinolin, Chinazolin, Chinoxalin, Triäthanolamin, Triamylamin, Tri-n-butylamin, Trifurfurylamin, Trihexylamin, N-Methylimidazol, N-Methylpyrrol, N-Äthylpiperidin, N-Methylpyrrolidin, Pyrazin, Pyrimidin, Acridin, Phenanthridin, Phenazin, N-Dimethylcyclohexylamin oder n-Propyl-diisopropylamin.

Bei der Umsetzung der reaktiven Amidacetale XIII oder der Ketoverbindung XIV genügt das Rühren der Ausgangsstoffe, ggf. unter Entfernung des abgespaltenen Alkohols bzw. des Reaktionswassers, durch Destillation bzw. Erwärmen an einem Wasserabscheider.

Die Reaktionstemperatur liegt normalerweise bei 0 - 120°C, vorzugsweise bei 20 - 80°C.

Üblicherweise werden die Komponenten in etwa stöchiometrischen Mengen eingesetzt, jedoch kann auch ein Überschuß einer der Komponenten, z. B. im Hinblick auf eine möglichst vollständige Umsetzung der anderen Komponente, vorteilhaft sein.

Setzt man gezielt einen Überschuß der Ausgangsstoffe XI - XII ein, mindestens aber 2 Equivalente pro Mol Ausgangsstoff VIIIf, so kommt es zu einer Substitution beider Aminowasserstoffe.

### Verfahren D:

Umsetzung eines l-(Pyridyl-2)-pyrazols der Formel VIIIe', in der Z, R² bis R⁴ und R⁶ die vorgenannte Bedeutung haben, mit elektrophilen Agenzien der Formel XV, in der B für eine elektronenziehende Abgangsgruppe steht gemäß DE 3520 330, JO 2142 - 785 oder EP 201 852:

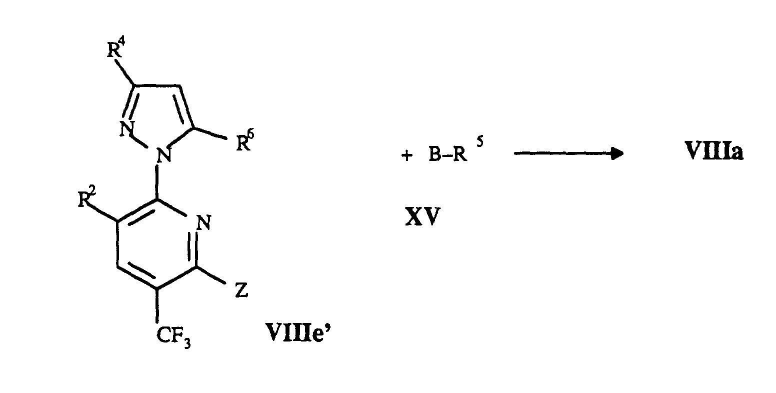

Hierbei steht der Rest R⁵ in der allgemeinen Formel XV vorzugsweise für Chlor, Brom, Nitro, Rhodano, Formyl, Alkanoyl mit 1 - 4 Kohlenstoffatomen im Alkylteil, C₁-C₄-Alkylsulfonyl, -sulfinyl oder -sulfenyl oder Halogenmethylsulfenyl.

B steht vorzugsweise für Halogen, insbesonderen Chlor oder Brom, für Hydroxy, für Akyl- oder Arylsulfonyloxy, für Alkanoyloxy oder Aroyloxy. Weitere elektrophile Agenzien sind Sulfurylchlorid, Phosphoroxichlorid / Dimethylformamid, Nitriersäure und andere üblicherweise zur elektrophilen Substitution verwendbare Stoffe.

Üblicherweise arbeitet man in einem der vorgenannten inerten Lösungs- oder Verdünnungsmittel.

Die Reaktionstemperatur liegt normalerweise bei 0 - 150°C, vorzugsweise 10 - 110°C.

### Verfahren E:

Diazotierung eines 1-(Pyridyl-2)-5-aminopyrazols der Formel VIIIf, in der Z, R², R⁴ und R⁵ die vorgenannte Bedeutung besitzen und anschließende Verkochung zu der entsprechenden 5-Hydroxyverbindung nach Hoben-Weyl, "Methoden der organischen Chemie" IV. Auflage, Bd. 6/1C, S. 247, Georg Thieme Verlag, Stuttgart 1968, oder Umsetzung zu den entsprechenden Fluorverbindungen ebenda Bd. 5/3, S. 213, Umsetzung unter Sandmeyerbedingungen zu den entsprechenden

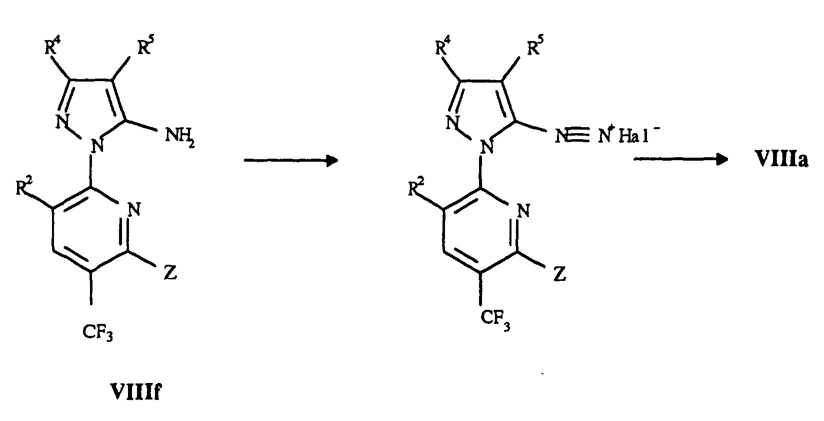

Chlorverbindungen, ebenda Bd. 5/3, S. 846, Bromverbindungen, ebenda Bd. 5/4, S. 437 oder Jodverbindungen, ebenda Bd. 5/4, S. 639, oder Umsetzung zu den entsprechenden Cyanoverbindungen, ebenda Bd. 8, S. 311 oder Rhodanoverbindungen, ebenda Bd. 9, S. 863, oder Umsetzung unter Meerweinbedingungen zu den entsprechenden Sufonsäurechloriden, ebenda Bd. 9, S. 579 und Folgereaktion mit Ammoniak oder Aminen nach Art der Schotten-Baumann-Reaktion, ebenda Bd 9, S. 609 oder Umsetzung mit Mercaptanen zu den entsprechenden Thioethern, ebenda Bd. 9, S. 116 oder mit Alkoholen zu den entsprechendne Ethern, ebenda. Bd. 6/3, S. 81.

### Verfahren F:

Umsetzung eines 1-(Pyridyl-2)-5-halogeno-pyrazols der Formel VIIIg, in der Halogen, Z, R², R⁴ und R⁵ die vorgenannte Bedeutung besitzen, mit Aminen der Formel XVI, in der R¹⁰ und R¹¹ die 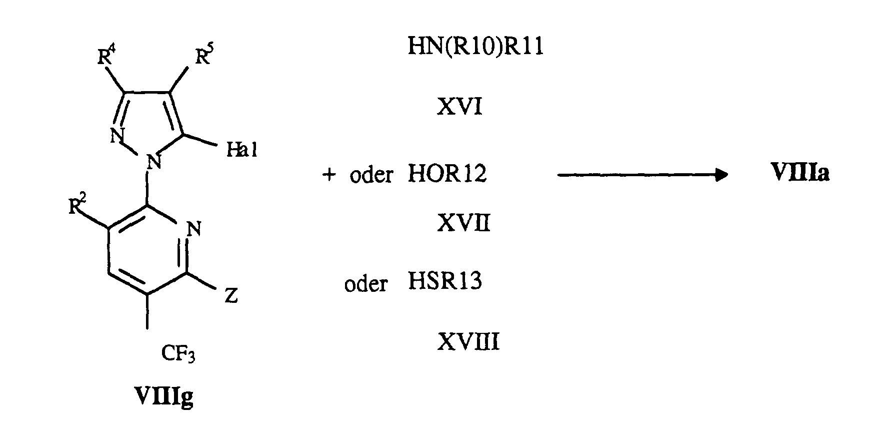 vorgenannten Bedeutungen besitzen, mit Alkoholen der Formel XVII in der R¹² die vorgenannte Bedeutung besitzt sowie Mercaptanen der Formel XVIII, in der R¹³ die vorgenannte Bedeutung besitzt, nach analogem Verfahren.

Zur Durchführung des Verfahrens setzt man pro Mol 5-Halogenopyrazol VIIIh im allgemeinen 1,0 - 5,0 mol, vorzugsweise 1,0 - 2,0 mol der Nucleophile XVI - XVIII ein. Die Reaktionsdurchführung, Aufarbeitung und Isolierung der Reaktionsprodukte I erfolgt nach allgemein üblichen Verfahren.

Als Verdünnungsmittel zur Durchführung des Verfahrens F kommen inerte organische Lösungsmittel infrage. Vorzugsweise verwendet man aliphatische oder aromatische, ggf. halogenierte Kohlenwasserstoffe, wie Pentan, Hexan, Heptan, Cyclohexan, Petrolether, Toluol, Xylol, Methylenchlorid, 1,2-Dichlorethan, Chlorbenzol oder Dichlorbenzol, Ether wie Diethylether, Diisopropylether, Methyl-tert.-butylether, Ethylenglykoldimethylether, Tetrahydrofuran oder Dioxan, Ester wie Essigsäureethylester, Nitrile wie Acetonitril oder Propionitril oder Amide wie Dimethylformamid, Dimethylacetamid oder N-Methylpyrrolidon.

Schließlich kann man jedoch auch die als Reaktionskomponenten verwendeten Nucleophile der Formel XVI - XVIII in entsprechendem Überschuß gleichzeitig als Verdünnungsmittel einsetzen.

Das Verfahren kann ggf. in Gegenwart eines basischen Katalysators zur Entfernung des entstehenden Halogenwasserstoffs durchgeführt werden. Als solche kommen alle üblichen anorganischen oder organischen Basen in Frage. Vorzugsweise verwendet man die bei Verfahren C genannten Basen.

Man kann jedoch auch direkt die Alkali- oder Erdalkalisalze der Nucleophile XVI - XVIII einsetzen, vorzugsweise die Lithium-, Natrium-, Kalium-, Magnesium- oder Calciumsalze.

Die Temperaturen können bei dem Verfahren F in einem größeren Bereich variiert werden. Im allgemeinen arbeitet man zwischen 0 und 150°C vorzugsweise zwischen 20 und 100°C.

Sofern nicht anders angegeben, werden alle vorstehend beschriebenen Verfahren zweckmäßigerweise bei Atmosphärendruck oder unter dem Eigendruck des jeweiligen Reaktionsgemisches vorgenommen.

Die Aufarbeitung der Reaktionsgemische erfolgt in der Regel nach an sich bekannten Methoden, beispielsweise durch Entfernen des Lösungsmittels, Verteilen des Rückstandes in einem Gemisch aus Wasser und einem geeigneten organischen Lösungsmittel und Aufarbeiten der organischen Phase auf das Produkt hin.

1-(Pyridyl)-pyrazole VIII mit CH-aciden Substituenten lassen sich auf an sich bekannte Weise in ihre Salze, vorzugsweise in ihre Alkalimetallsalze, überführen.

Salze von VIII, deren Metallion kein Alkalimetallion ist, können durch Umsalzen des entsprechenden Alkalimetallsalzes in üblicher Weise hergestellt werden, ebenso Ammonium- und Phosphoniumsalze mittels Ammoniak, Phosphonium-, Sulfonium- oder Sulfoxoniumhydroxiden.

Verbindungen VIII, die eine endständige Aminogruppe tragen, könne ferner Säureadditionssalze bilden. Geeignet sind allgemein die Salze von solchen Säuren, welche die herbizide Wirkung von VIII ebenfalls nicht negativ beeinträchtigen, also z. B. die Hydrochloride und -bromide, Sulfate, Nitrate, Phosphate, Oxalate oder die Dodecylbenzolsulfonate.

### Nicht erfindungsgemäße Beispiele

### Beispiel I.1.

### Umsetzung von 2-Amino-3,6-dichlor-5-trifluormethylpyridin mit Natriummethylat.

2,6 g (0,014 mol) 30 %iges Natriummethylat wurden bei 20°C innerhalb 5 Minuten unter Rühren zu einer Mischung von 3,3 g(0,014 mol) 2-Amino-3,6-dichlor-5-trifluormethylpyridin in 100 ml Methanol gegeben. Unter HPLC-Kontrolle wurde zunächst 1 Stunde bei 30°C und dann 6 Stunden unter Rückfluß gerührt, wobei im wesentlichen das Ausgangsmaterial erhalten blieb. Nach dem Abkühlen wurde das Reaktionsgemisch in Vakuum eingeengt, in Methylenchlorid aufgenommen und mit Wasser extrahiert. Die organische Phase ergab nach dem Trocknen über Magnesiumsulfat und Einengen 3,2 g (97 % d. Th.) verunreinigtes Ausgangsmaterial zurück.

### Beispiel I.2.

### 2-Amino-3-chlor-6-methoxy-pyridin-5-carbonsäuremethylester

4,0 g (0,0173 mol) 2-Amino-3,6-dichlor-5-trifluormethylpyridin in 20 ml Methanol wurden innerhalb 5 Minuten unter Rühren bei 10°C zu einer Mischung von 5,5 g (0,0866 mol) 88%igen Kaliumhydroxidpulver in 80 ml Methanol gegeben. Unter HPLC-Kontrolle wurde das Reaktionsgemisch 10 Stunden unter Rückfluß gerührt. Nach Zugabe von weiteren 1,1 g (0,0173 mol) 88%igem Kaliumhydroxidpulver und Rückflußkochen während 2 Stunden wurde abgekühlt und eingeengt. Der Rückstand wurde in Methyl-tert.-butylether aufgenommen und mit Wasser extrahiert. Nach dem Trocknen und Einengen erhielt man 4,6 g Harz, das in Pentan verrührt, abgesaugt und dann getrocknet wurde. Eine anschließende Chromatograhie über Kieselgel mit Methylenchlorid lieferte in den Fraktionen 15 - 50 2,3 g (61 %, d. Th.) der Titelverbindung von Fp 162-164°C. Die Fraktionen 51 - 65 enthielten 0,3 g (6,5 % d. Th.) 2-Amino-3,6-dichlor-5-(trimethoxymethyl)-pyridin vom Fp 99 - 107°C.

### Beispiel I.3.

### 2-Amino-3-chlor-6-methoxy-5-trifluormethylpyridin

6,7 g (0,029 mol) 2-Amino-3,6-dichlor-5-trifluormethylpyridin wurden innerhalb 5 Minunten bei 20°C unter Rühren zu einer Mischung von 2,4 g (0,0377 mol) 88%igem Kaliumhydroxidpulver in 10 ml Methanol und 100 ml Dimethylformamid gegeben. Unter HPLC-Kontrolle wurde das Reaktionsgemisch nun 89 Stunden bei 130°C gerührt, wobei nach 40 Stunden weitere 5 ml Methanol der Reaktionsmischung zugesetzt wurden. Nach der Aufarbeitung gemäß Beispiel I.2. erhielt man in den Fraktionen 3 und 4 0,3 g (4,6 % d. Th.)der Titelverbindung mit η_{D}²⁴ = 1.5143. Die Fraktion 5 enthielt 0,7 g (10 % d. Th.) 2-Amino-3-chlor-6-dimethylamino-5-trifluormethylpyridin (NMR 400 MHz, CDCl₃ 7,58 (Pyridin), 2,95 (CH₃)₂N und die Fraktionen 9 - 16 1,1 g (16 % d. Th.) Ausgangsmaterial. Weitere Nebenkomponenten waren in den folgenden Fraktionen enthalten.

### Beispiel I.4.

### Umsetzung von 2,5-Dichlor-6-methoxy-3-trifluormethylpyridin mit Ammoniak.

### a) Drucklos

6 g (0,353 mol) gasförmiges Ammoniak wurden innerhalb 8 Stunden bei 80 - 90°C unter Rühren in eine Mischung von 10 g (0,041 mol) 3,6-Dichlor-2-methoxy-5-trifluormethylpyridin in 100 ml Propanol eingegast. Nach der HPLC-Untersuchung trat hierbei keine Änderung in der Zusammensetzung des Reaktionsgemisches ein. Auch das Verteilen der Reaktionsmischung in Wasser / Methyl-tert.-butylether, Trocknen und Einengen der organischen Phase lieferte nach dem NMR-Spektrum ca. 95 % reines, öliges Ausgangsmaterial zurück.

### b) unter Druck

12,4 g (0,73 mol) gasförmiges Ammoniak wurden bei 140°C unter Rühren in eine Mischung von 12,8 g (0,052 mol) 3,6-Dichlor2-methoxy-5-trifluormethylpyridin in 100 ml Methanol in einem Autoklaven eingepreßt, wobei der Druck bis auf 200 bar anstieg. Eine Probe zeigte nach einer halben Stunden kein Ausgangsmaterial mehr. Die Reaktionslösung wurde eingeengt und zwischen Methylenchlorid und Wasser verteilt. Nach dem Trocknen, Einengen, Verreiben des Rückstandes mit n-Pentan, Absaugen und Trocknen erhielt man 3,4 g (28 % d. Th.) 2-Amino-3,6-dichlor-5-trifluormethylpyridin vom Fp 110 - 112°C. Das eingeengte Pentanfiltrat (5 g) bestand nach der HPLC-Analyse aus weiteren 19 % 2-Amino-3,6-dichlor5-trifluormethylpyridin sowie 5 weiteren Verbindungen.

### Erfindungsgemäße Beispiele

### Synthese der Vorprodukte

### Beispiel II.1

3-Chlor-2,6-difluor-5-trifluormethylpyridin 110,3 g (0,44 mol) 2,3,6-Trichlor-5-trifluormethylpyridin und 58,7 g (1,01 mol) Kaliumfluorid wurden unter Rühren zu 500 ml Sulfolan (gemäß S. 13 mit Thionylchlorid getrocknet) gegeben und unter HPLC-Kontrolle 2 1/2 Stunden bei 150 - 160°C gerührt. Nach dem Abkühlen auf 50°C wurden aus dem Reaktionsgemisch 93 g (98 % d. Th.) der Titelverbindung bei 40 - 45°C, 25 mbar abdestilliert. NMR (400 MHz, CDCl₃) δ 8,2 Pyr-H/t; η_{D}²³ = 1,4229.

### Beispiel II.2

### 2-Amino-3-chlor-6-fluor-5-trifluormethylpyridin

12,5 g (0,74 mol) gasförmiges Ammoniak wurden innerhalb 45 Minuten unter Rühren in eine Mischung von 40 g (0,184 mol) 3-Chlor-2,6-difluor-5-trifluormethylpyridin in 200 ml Ethanol bei 50 - 60°C eingegast. Nach 1 1/2 Stunden Rühren bei 55°C wurde das Reaktionsgemisch abgekühlt und eingeengt. Der Rückstand wurde in Wasser verrührt, abgesaugt und getrocknet, wobei man 36,7 g (93 % d. Th.) der Titelverbindung von Fp 101 - 103°C erhielt.

### Beispiel II.3

### 3-Chlor-6-fluor-2-hydrazino-5-trifluormethylpyridin

10,5 g (0,21 mol) Hydrazinhydrat wurden bei 50°C unter Rühren innerhalb 15 Minuten zu einer Mischung von 21,8 g(0,1 mol) 3-Chlor-2,6-difluor-5-trifluormethylpyridin in 100 ml Propanol gegeben und 2 Stunden bei gleicher Temperatur nachgerührt. Nach dem Abkühlen wurde der Reaktionsansatz auf 1,5 l Wasser gegossen und der ausgefallene Niederschlag abgesaugt. Er wurde zur Reinigung in Essigester aufgenommen und mit Wasser extrahiert. Nach dem Trocknen und Einengen erhielt man 20,9 g (91 % d. Th.) der Titelverbindung von Fp 104 - 106°C; eine sublimierte Probe schmolz bei 111 - 113°C.

### Synthese der Endprodukte

### Beispiel III.1

2-Amino-3-chlor-6-methoxy-5-trifluormethylpyridin 4,6 g (0,0256 mol) 30 %ige Natriummethylatlösung wurden innerhalb 5 Minuten unter Rühren bei 22 - 27°C zu einer Mischung von 5,5 g (0,0256 mol) 2-Amino-3-chlor-6-fluor-5-trifluormethylpyridin in 80 ml Methanol gegeben. Nach 6 Stunden Rühren unter Rückfluß wurde das Reakionsgemisch abgekühlt, eingeengt und in Methlenchlorid / Wasser verteilt. Die organische Phase wurde abgetrennt, getrocknet und über eine Säule mit Kieselgel chromatographiert, wobei man 5,0 g (86 % d. Th.) der Titelverbindung mit η_{D}²⁴ = 1,5143 erhielt. NMR (400 MHz, CDCl₃) δ 7,6, Pyr (1/s); 3,91, CH₃O (3/s) (Tabelle I, Nr. 1.001).

### Beispiel III.2

2-Amino-3-chlor-6-propargyloxy-5-trifluormethylpyridin 3,9 g (0,153 mol) Natriumhydrid wurden unter Stickstoff unter Rühren in 250 ml Methyl-tert.-butylether vorgelegt und dann während 20 Minuten bei 25 - 30°C unter Rühren tropfenweise mit 20 ml Propargylalkohol versetzt. Nach 30 Minuten Rühren bei 25°C wurden 32,9 g (0,153 mol) 2-Amino-3-chlor-6-fluor-5-trifluormethylpyridin in 100 ml Methyl-tert.-butylether innerhalb 10 Minuten zugegeben und 14 Stunden bei 25°C gerührt. Das Reaktionsgemisch wurde mit Wasser extrahiert, getrocknet und eingeengt, wobei man 37,8 (99 % d. Th.) der Titelverbindung als farbloses Öl mit η_{D}²⁴ = 1,5217 m erhielt (Tabelle I, Nr. 1.035).

In den folgenden Tabellen sind noch weitere Verbindungen I aufgeführt, die nach einem der beschriebenen Verfahren hergestellt wurden oder herstellbar sind.

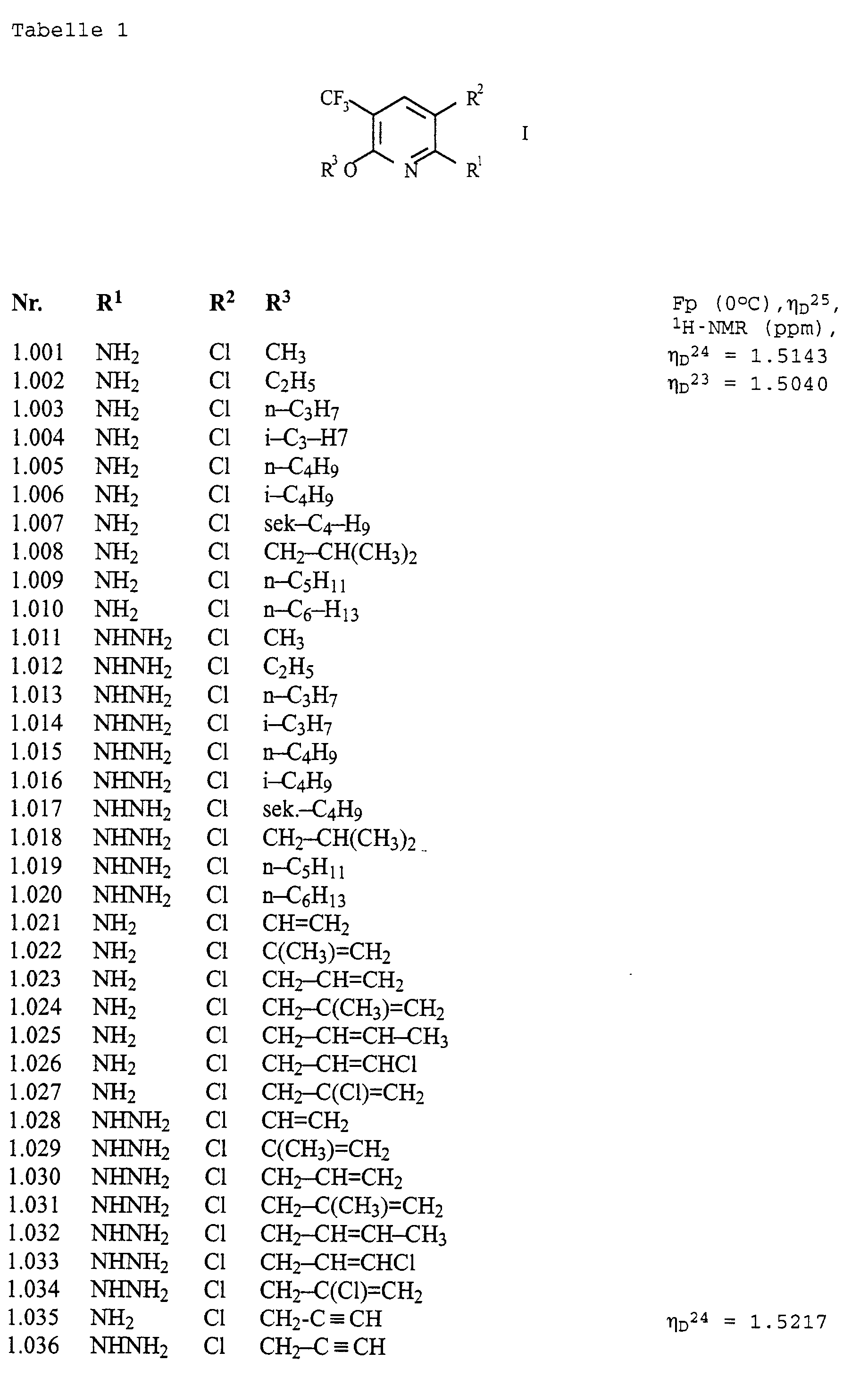

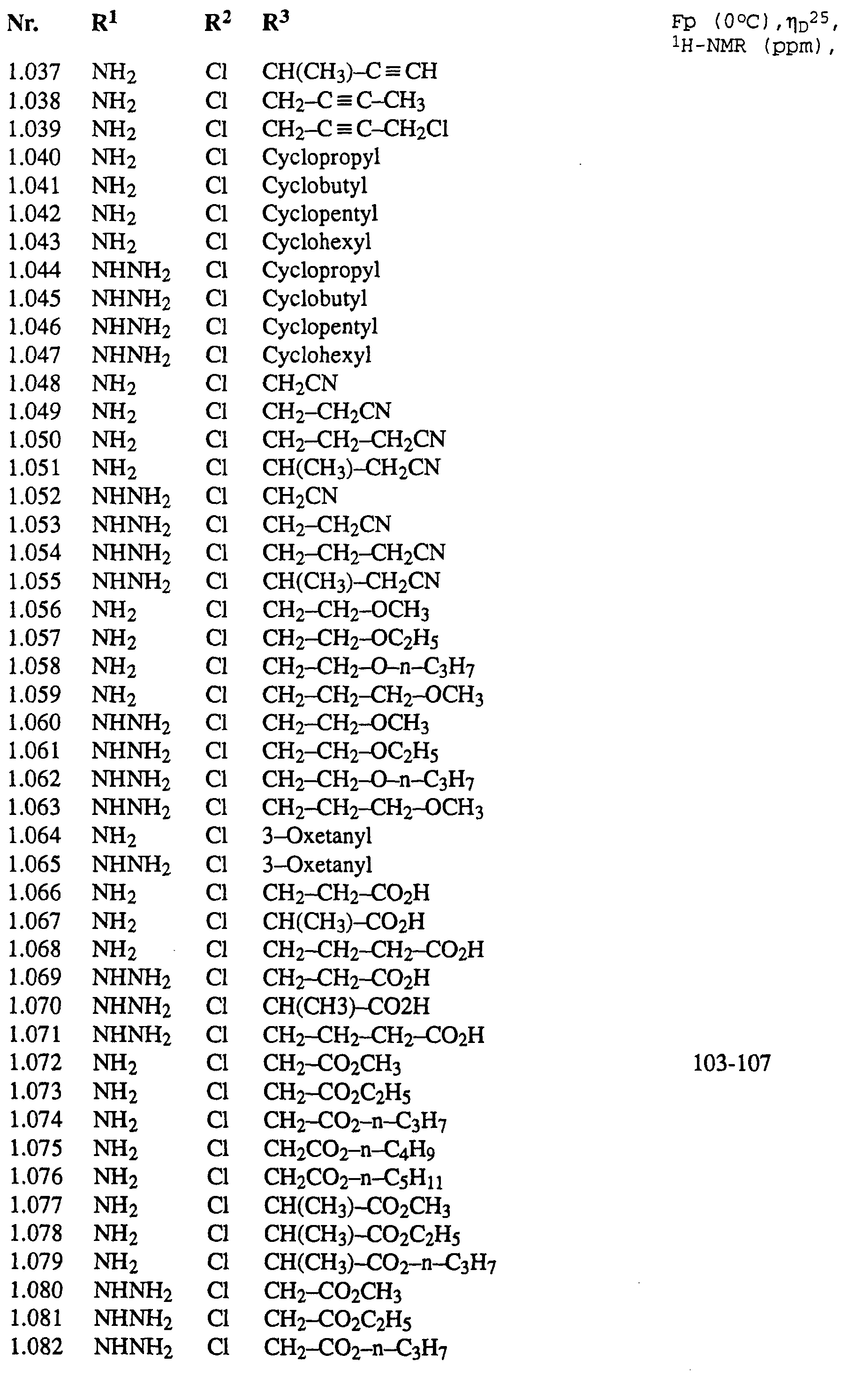

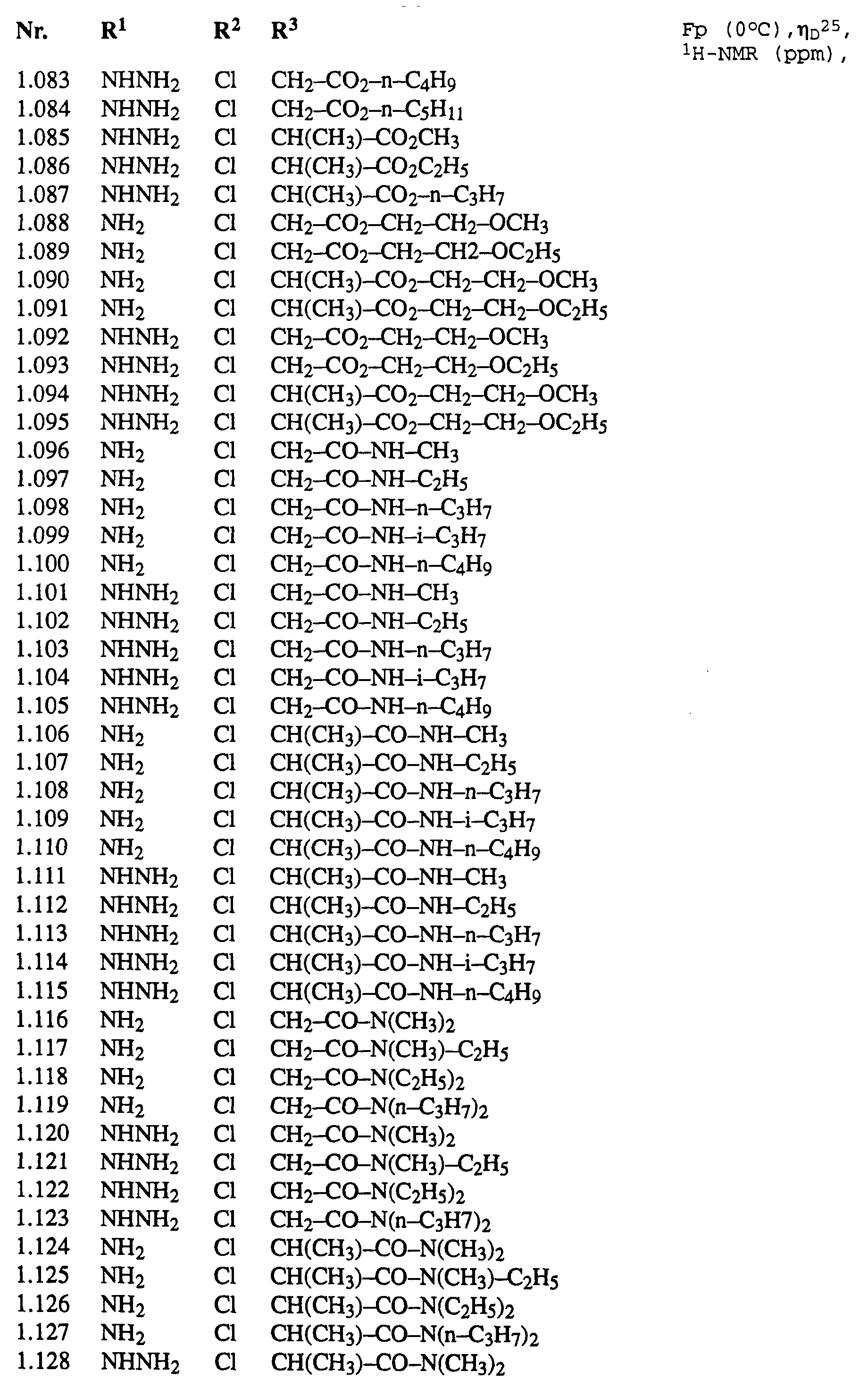

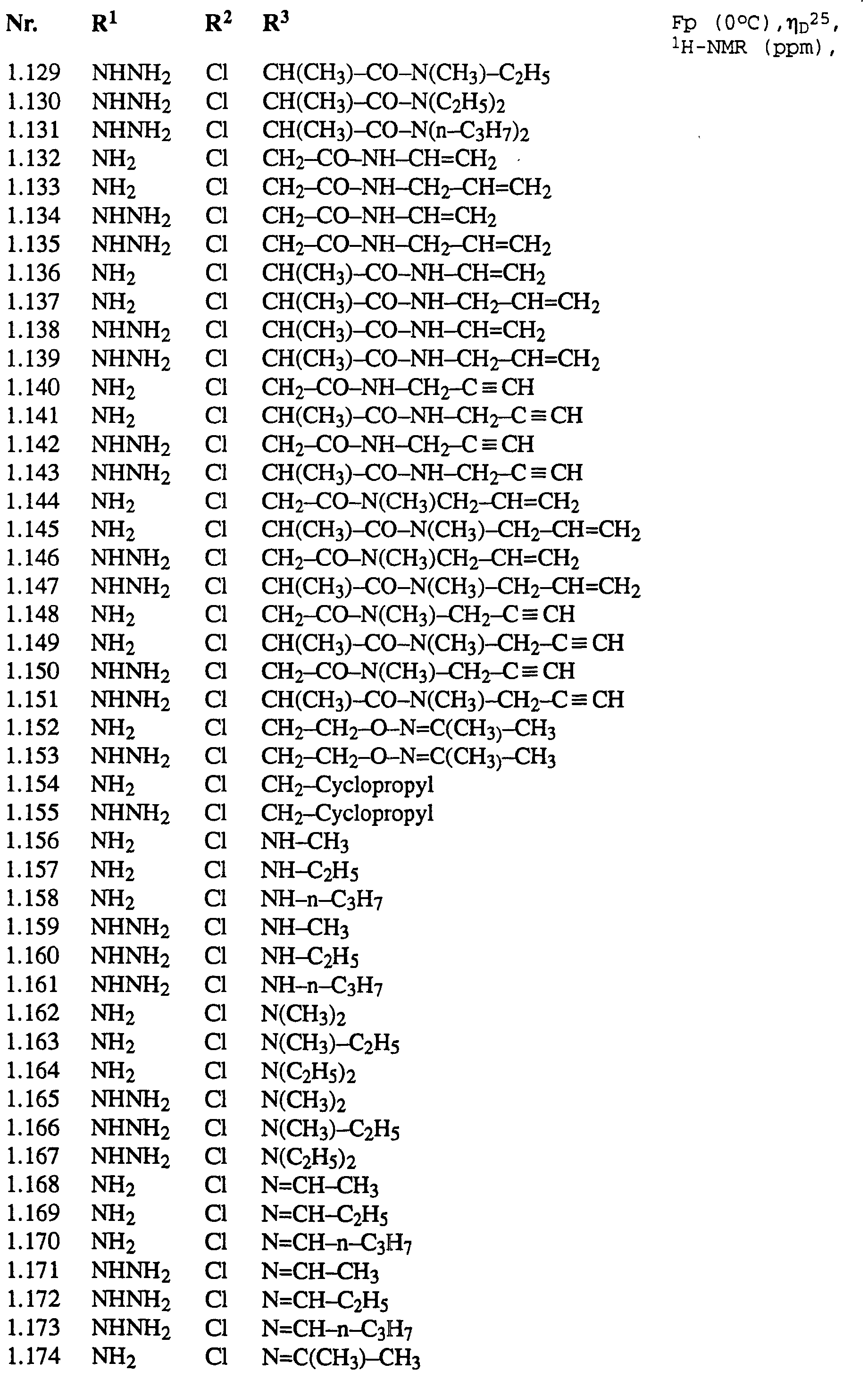

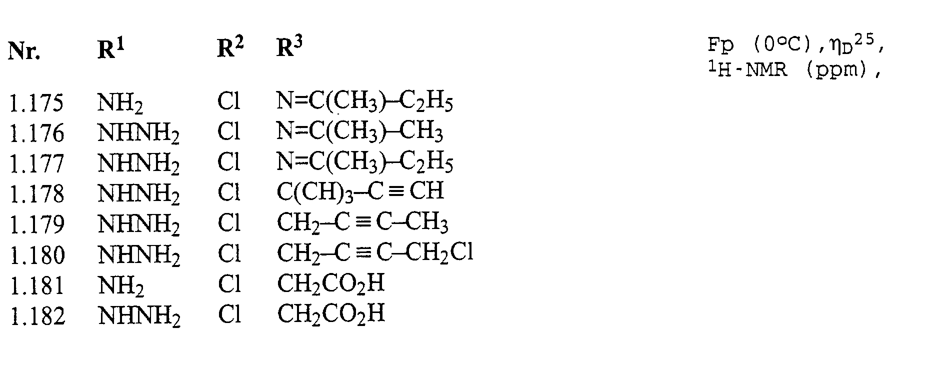

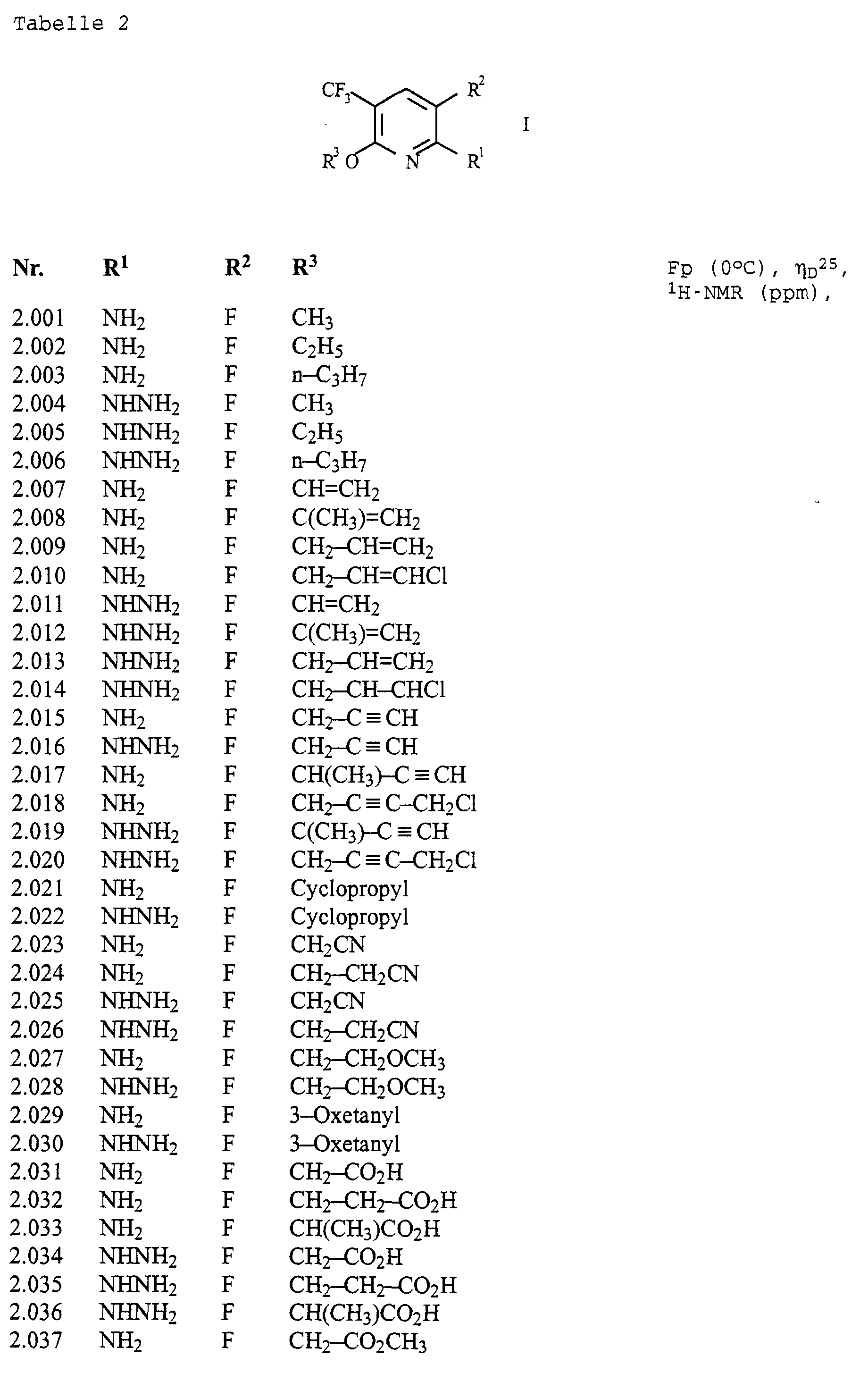

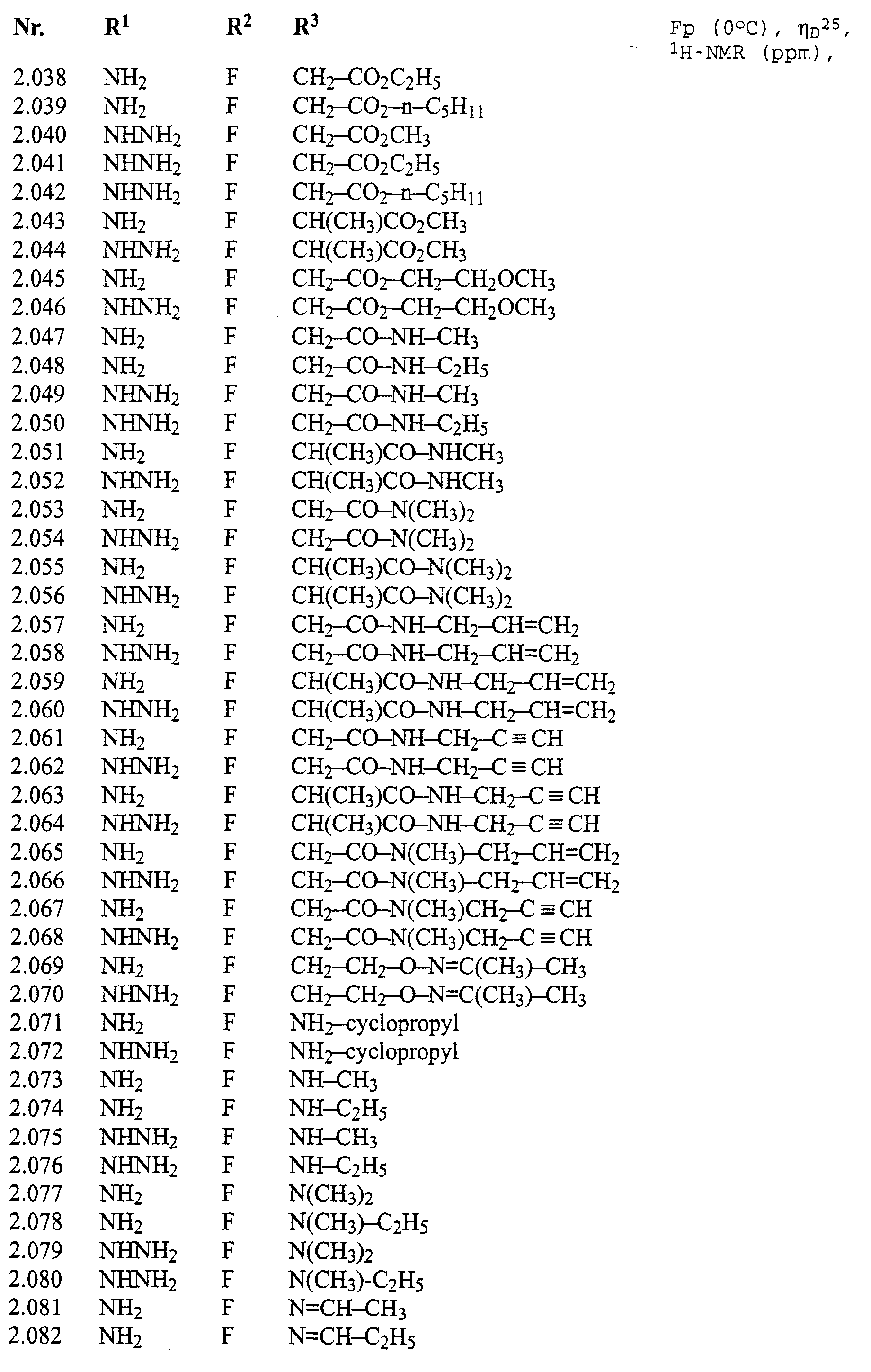

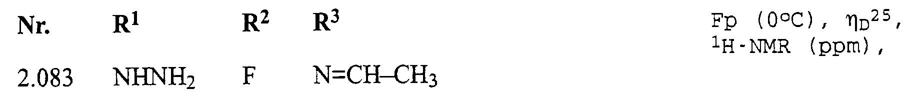

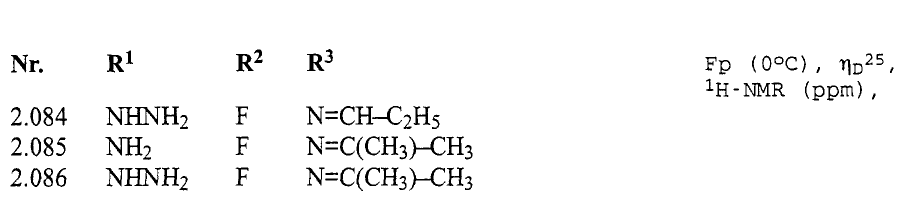

### Beispiele für 1-(Pyridyl)-Pyrazole:

### Beispiel IV.I.

### 5-Amino-4-cyano-1-[3-chlor-6-fluor-5-trifluormethyl-pyridyl-2] -pyrazol

12,2 g [0,1 mol] Ethoxymethylen-malonsäuredinitril wurden innerhalb 5 Minuten unter Rühren bei 22°C zu 23 g [0,1 mol] 3-Chlor-6-fluor-2-hydrazino-5-trifluormethyl-pyridin in 200 ml Ethanol gegeben und 8 Stunden bei 78°C gerührt. Der ausgefallene Niederschlag wurde abgesaugt und zwischen Wasser/Methylendchlorid verteilt. Die organische Phase wurde über Magnesiumsulfat getrocknet und über neutrales Aluminiumoxid gesaugt. Das Filtrat wurde eingeengt, mit Ether/Pentan 1:1 verrührt, abgesaugt und getrocknet, wobei man 12,6 g [41 % d.Th] der Titelverbindung von Fp. 156-159°C erhielt [Wirkstoffbeispiel Nr. 3.019].

### Beispiel IV.2.

### 5-Amino-4-cyano-1-(3-chlor-6-methoxy-5-trifluormethyl-pyridyl-2)-pyrazol

5 ml Methanol wurden innerhalb 10 Minuten bei 20°C zu einer Suspension von 0,23 g (0,0092 mol) 95 %igem Natriumhydrid in 75 ml Methyl-tert.-butylether gegeben und 25 Minuten bis zu einer klaren Lösung gerührt. Anschließend wurden 3 g (0,0098 mol) 5-Amino-4-cyano-1-(3-chlor-6-fluor-5-trifluormethyl-pyridyl-2)-pyrazol (Beispiel IV.I.) in 50 ml Methyl-tert.-butylether unter Rühren zugegeben und 2 Stunden bei 25°C gerührt. Es wurden ca. 50 g Eis und 100 ml in Salzsäure zugegeben und die Phasen getrennt. Die organische Phase wurde mit gesättigter Kochsalzlösung gewaschen, getrocknet und über Kieselgel filtriert. Nach dem Einengen, Verrühren mit Ether/ Pentan, Absaugen und Trocknen erhielt man 2,4 g (77 % d. Th.) der Titelverbindung vom Fp 135 - 139°C (Wirkstoffbeispiel Nr. 3.003).

In den folgenden Tabellen sind weitere Verbindungen VIII aufgeführt, die analog den Beispielen hergestellt wurden oder entweder nach den vorstehend beschriebenen Verfahren oder nach an sich bekannten Methoden herstellbar sind.

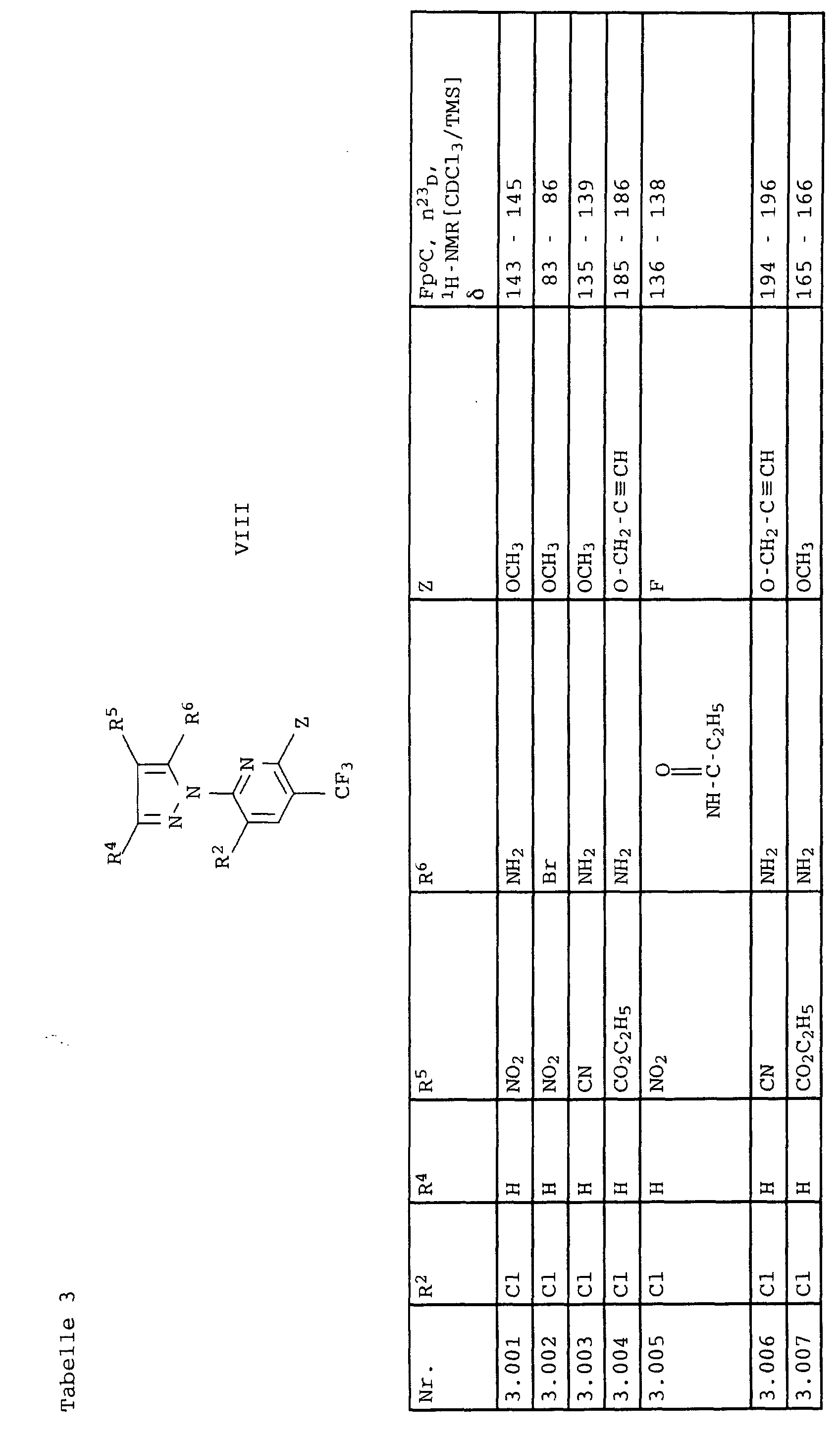

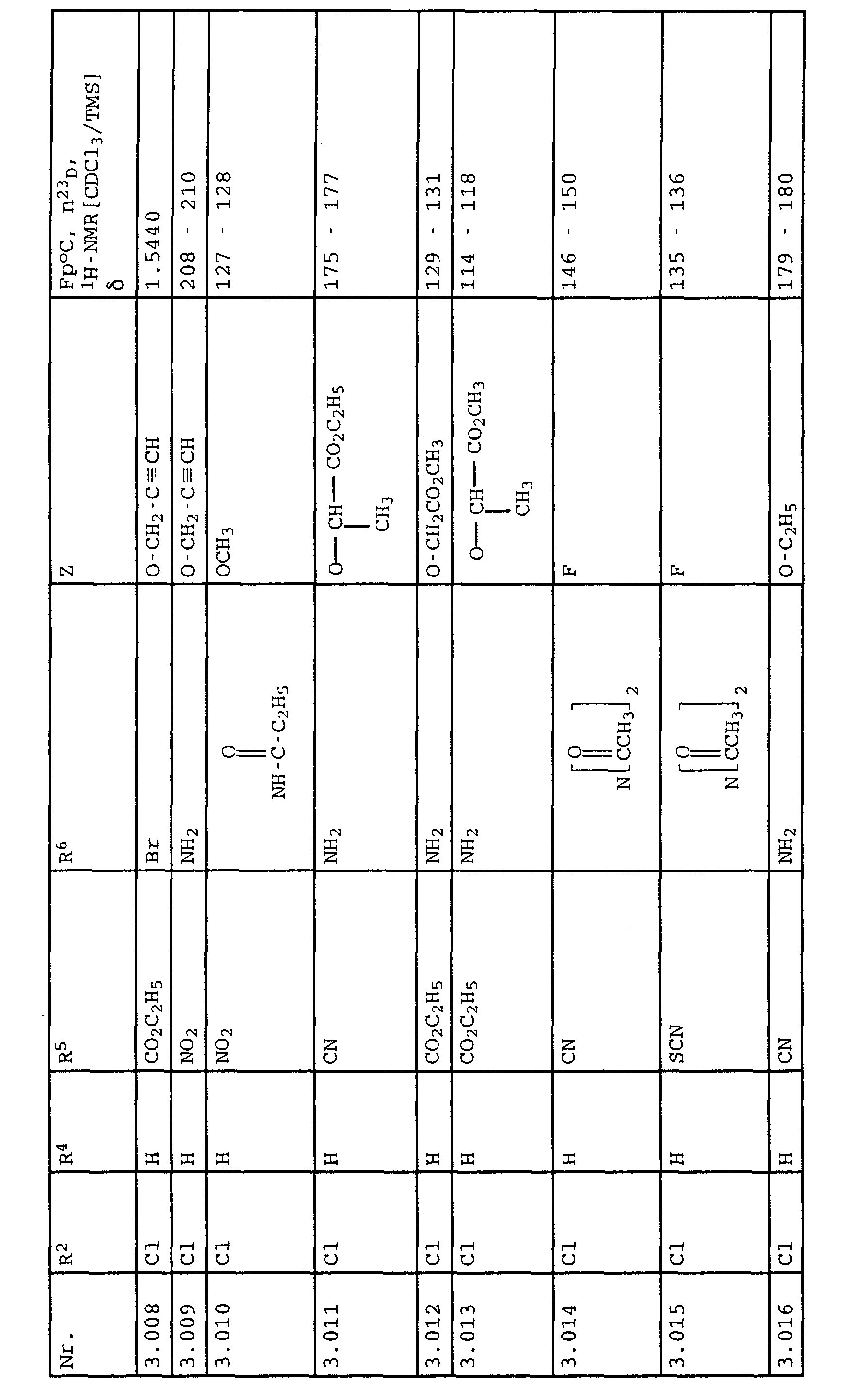

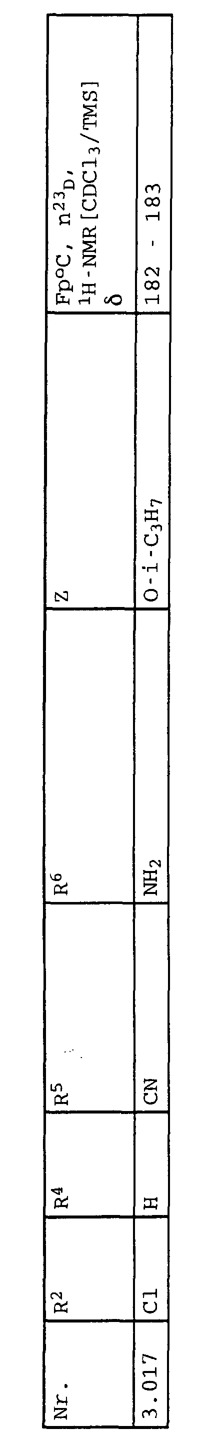

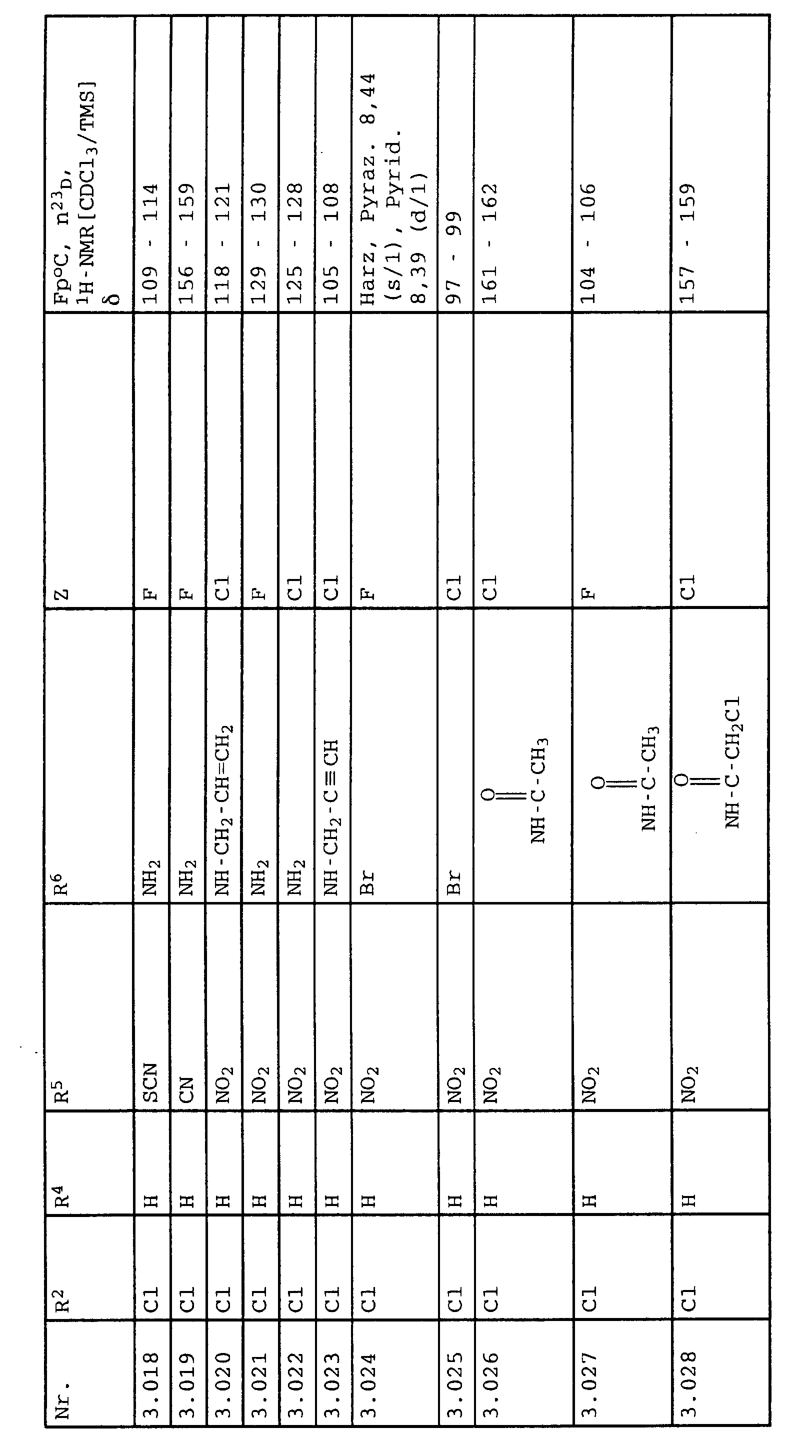

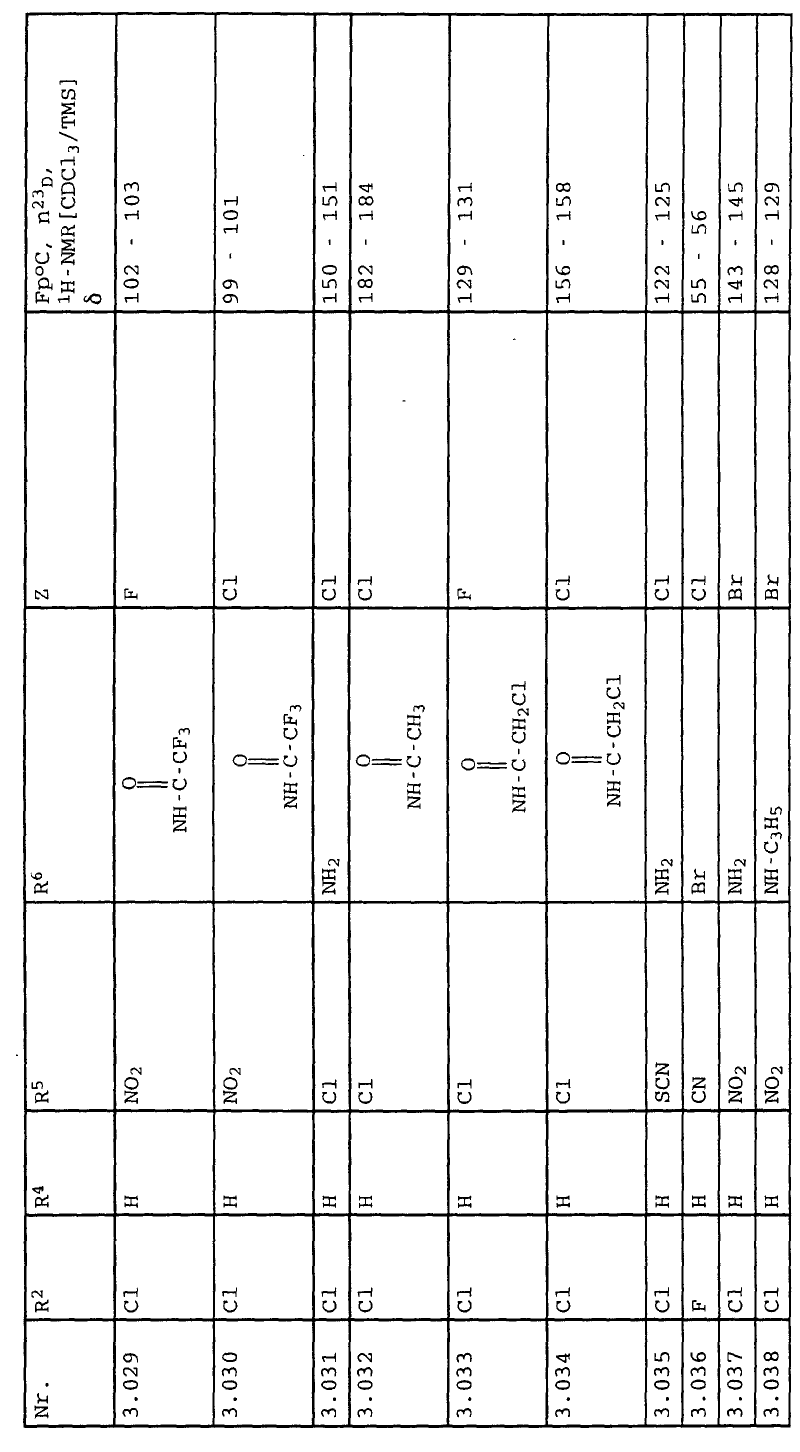

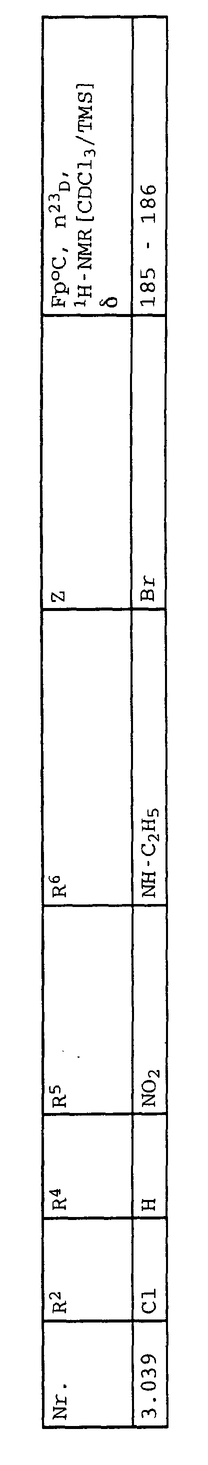

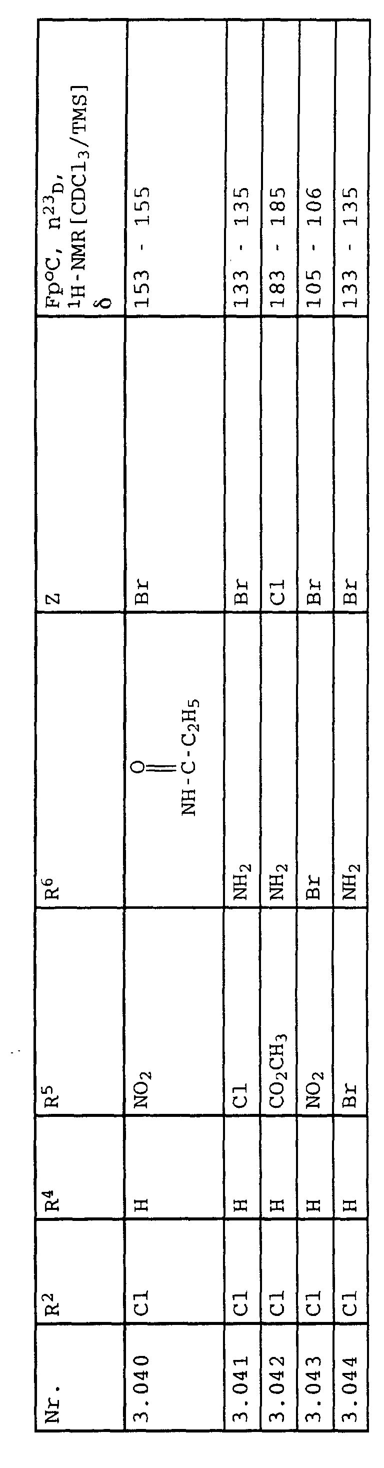

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulagris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays,

Darüber hinaus können die Verbindungen I auch in Kulturen, die durch Züchtung einschließlich gentechnischer Methoden gegen die Wirkung von Herbiziden tolerant sind, verwandt werden.
Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew. %, vorzugsweise 0,01 bis 95 Gew. %, Wirkstoff. Die Wirkstoffe werden dabei in einer Reinheit von 90% bis 100%, vorzugsweise 95% bis 100% (nach NMR-Sektrum) eingesetzt.

Die erfindungsgemäßen Verbindungen I können beispielsweise wie folgt formuliert werden:
- I: 20 Gewichtsteile der Verbindung Nr. 3.001 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
- II: 20 Gewichtsteile der Verbindung Nr. 3.002 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
- III: 20 Gewichtsteile des Wirkstoffs Nr. 3.003 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
- IV: 20 Gewichtsteile des Wirkstoffs Nr. 3.005 werden mit 3 Ge-wichtsteilen des Natriumsalzes der Diisobutylnaphthalin-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew. % des Wirkstoffs enthält.
- V: 3 Gewichtsteile des Wirkstoffs Nr. 3.010 werden mit 97 Gewichtsteilen feinteiligem Kaolin vermischt. Man erhält auf diese Weise ein Stäubemittel, das 3 Gew. % des Wirkstoffs enthält.
- VI: 20 Gewichtsteile des Wirkstoffs Nr. 3.016 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondesates und 68 Gewichtsteilen eines paraffinischen Mineralöls innig vermischt. Man erhält eine stabile ölige Dispersion.
- VII: 1 Gewichtsteil der Verbindung Nr. 3.017 wird in einer Mischung gelöst, die aus 70 Gewichtsteilen Cyclohexanon, 20 Gewichtsteilen ethoxyliertem Isooctylphenol und 10 Ge-wichtsteilen ethoxyliertem Rizinusöl besteht. Man erhält ein stabiles Emulsionskonzentrat.
- VIII: 1 Gewichtsteil der Verbindung Nr. 3.018 wird in einer Mischung gelöst, die aus 80 Gewichtsteilen Cyclohexanon und 20 Gewichtsteilen Emulphor EL (ethoxyliertes Rizinusöl/casteroil) besteht. Man erhält ein stabiles Emulsionskonzentrat.

Zur Verbreiterung des Wirkungsspektrums und zur Erzielung synergistischer Effekte können die substituierten Trifluormethylpyridine I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF3-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a.S.).

### Anwendungsbeispiele

Die herbizide Wirkung der substituierten Trifluormethylpyridine der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung beträgt 0.0156 bzw. 0.0078 kg/ha a.S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Glycine max | Sojabohnen | soybeans |
| Abutilon theophrasti | Chinesischer Hanf | velvet leaf |
| Solanum nigrum | Schwarzer Nachtschatten | black nightshade |

### Vergleichsbeispiel

Nach den oben beschriebenen Methoden wurde die erfindungsgemäße Verbindung und das bekannte Vergleichsmittel im Gewächshaus im Nachauflaufverfahren eingesetzt.

### Das verwendete Vergleichsmittel ist:

- A: Aus DE 3 520 330

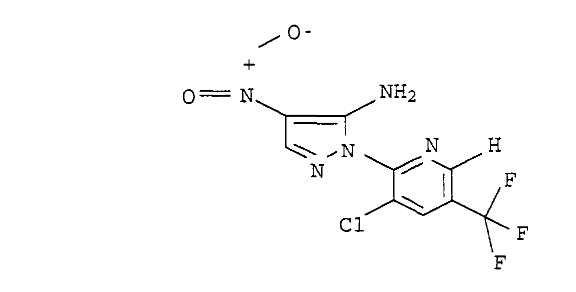

Die Werte der Tabelle 4 zeigen deutlich die Vorteile der erfindungsgemäßen Verbindung. Sie ist im Unterschied zum Vergleichsbeispiel A selektiv an der Kultur Soja bei gleichzeitig hoher herbizider Wirkung.

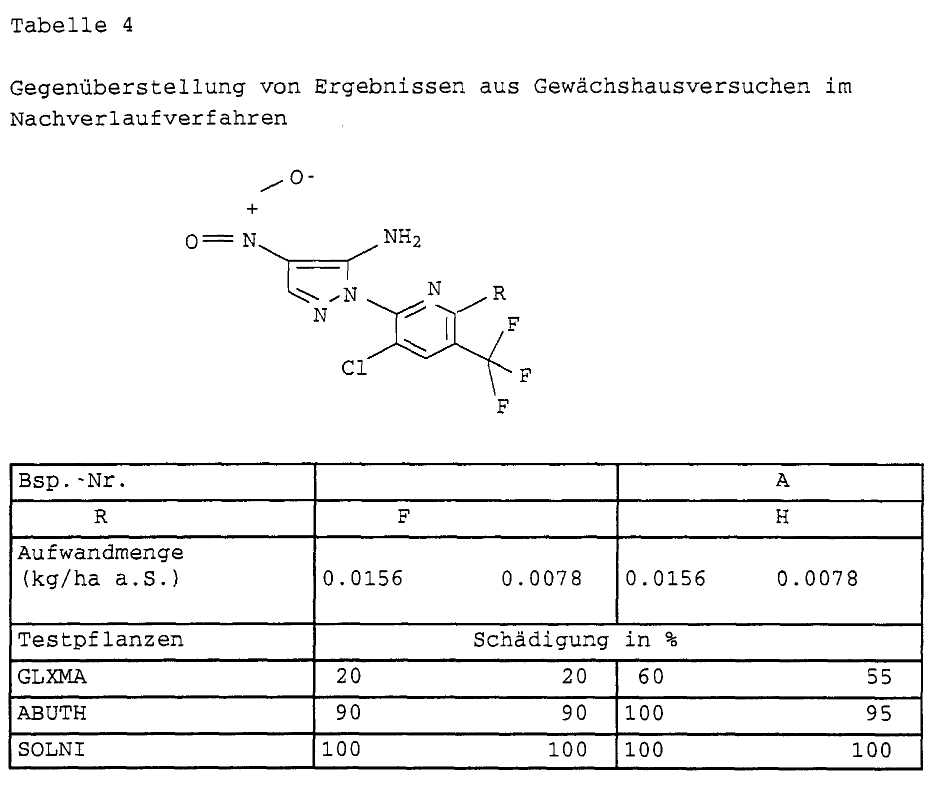

## Patentansprüche

1. Substituierte Trifluormethylpyridine der allgemeinen Formel I 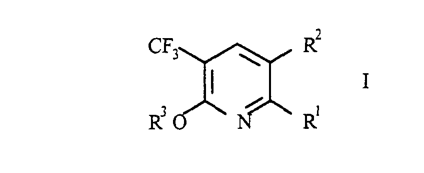 in der die Substituenten die folgende Bedeutung haben:
R¹ Amino oder Hydrazino,;
R² Halogen; und
R³ C₁-C₆-Alkyl, C₂-C₆-Alkenyl oder C₂-C₆-Alkinyl, wobei diese Gruppen bis zu 6 Halogenatome tragen können,
C₃-C₆-Cycloalkyl, das bis zu 3 C₁-C₃-Alkylreste tragen kann, C₁-C₆-Cyanoalkyl, C₁-C₄-Alkoxy-C₂-C₆-alkyl, 3-Oxetanyl, Carboxyl-C₁-C₆-alkyl, C₁-C₆-Alkoxycarbonyl-C₁-C₆-Alkyl, C₁-C₄-Alkoxy-C₂-C₄-alkoxycarbonyl-C₁-C₆-alkyl, Aminocarbonyl-C₁-C₆-alkyl, C₁-C₄-Alkylaminocarbonyl-C₁-C₆-alkyl, C₁-C₄-Dialkylaminocarbonyl-C₁-C₆-alkyl, C₂-C₄-AlkenylaminocarbonylC₁-C₆-alkyl, C₃-C₄-Alkinylaminocarbonyl-C₁-C₆-alkyl, C₁-C₄-Alkyl-C₃-C₄-alkenylaminocarbonyl-C₁-C₆-alkyl, C₁-C₄-Alkyl-C₃-C₄-alkinylaminocarbonyl-C₁-C₆-alkyl, C₃-C₆-(α-Alkylalkyliden)iminoxy-C₂-C₆-alkyl, Cyclopropylmethyl, C₁-C₆-Alkylamino, C₁-C₆-Dialkylamino, C₁-C₆-Alkylidenimino- oder α-(C₁-C₄-Alkyl)-C₂-C₆-alkylidenimino; und
Halogen, Fluor, Chlor, Brom oder Jod.

2. Substituierte Trifluormethylpyridine nach Anspruch 1, in denen
R¹ Amino oder Hydrazino,
R² Chlor oder Fluor
R³ C₁-C₄-Alkyl, C₂-C₄-Alkenyl, oder C₂-C₄-Alkinyl, wobei diese Gruppen bis zu 6 Halogenatome tragen können, C₃-C₆-Cycloalkyl, das seinerseits bis zu 3 Methylreste tragen kann, C₁-C₂-Alkoxy-C₂-C₄-alkoxycarbonyl-C₁-C₄-alkyl, C₁-C₄-Cyanoalkyl, C₁-C₂-Alkoxy-C₂-C₄-alkyl, 3-Oxetanyl, Carboxyl-C₁-C₄-alkyl, C₁-C₄-Alkoxycarbonyl-C₁-C₄-alkyl, C₁-C₂-Alkoxy-C₂-C₄-alkoxycarbonyl-C₁-C₄-alkyl, C₁-C₂-Alkylaminocarbonyl-C₁-C₄-alkyl, C₃-C₄-(α-Alkyliden)iminoxy-C₂-C₄-alkyl, Cyclopropylmethyl, C₁-C₄-Alkylamino, C₁-C₄-Dialkylamino, C₁-C₄-Alkylidenimino oder α-(C₁-C₂-Alkyl)-C₂-C₄-Alkylidenimino bedeuten.

3. Verfahren zur Herstellung von Trifluormethylpyridinen der allgemeinen Formel I 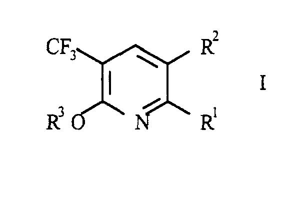 gemäß Anspruch 1, **dadurch gekennzeichnet, daß** man ein 3-Halogen-2,6-difluor-5-trifluormethyl-pyridin der Formel III 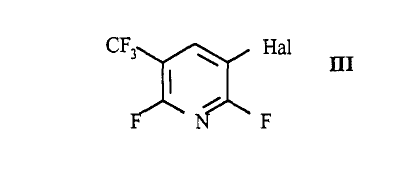 nacheinander mit Ammoniak oder Hydrazin zu dem 2-Amino- bzw. 2-Hydrazino-3-halogen-6-fluor-5-trifluormethyl-pyridin der Formel IV oder IV' 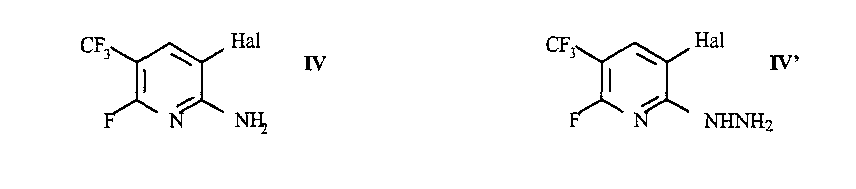 ggf. in Gegenwart einer organischen Hilfsbase und eines Lösungsmittels umsetzt und dieses dann mit einem Alkohol der allgemeinen Formel V
HOR³ V
oder dessen Alkali- oder Erdalkalisalz, ggf. in Gegenwart einer Base und eines Lösungsmittels zur Umsetzung bringt.

4. 2-Amino-3-chlor-6-methoxy-5-trifluormethyl-pyridin.

5. 2-Amino-3-chlor-6-propargyloxy-5-trifluormethyl-pyridin.

6. 2-Amino-3-chlor-6-methoxycarbonyl-methoxy-5-trifluormethylpyridin.

7. 2-Amino-3-chlor-6-ethoxy-5-trifluormethyl-pyridin.

## Claims

1. A substituted trifluoromethylpyridine of the general formula I 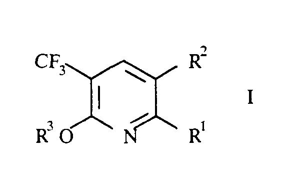 where the substituents have the following meanings:
R¹ is amino or hydrazino;
R² is halogen; and
R³ is C₁-C₆-alkyl, C₂-C₆-alkenyl or C₂-C₆-alkynyl, it being possible for these groups to have attached to them up to 6 halogen atoms,
C₃-C₆-cycloalkyl which can have attached to it up to 3 C₁-C₃-alkyl radicals, C₁-C₆-cyanoalkyl, C₁-C₄-alkoxy-C₂-C₆-alkyl, 3-oxetanyl, carboxyl-C₁-C₆-alkyl, C₁-C₆-alkoxycarbonyl-C₁-C₆-alkyl, C₁-C₄-alkoxy-C₂-C₄-alkoxycarbonyl-C₁-C₆-alkyl, aminocarbonyl-C₁-C₆-alkyl, C₁-C₄-alkylaminocarbonyl-C₁-C₆-alkyl, C₁-C₄-dialkylaminocarbonyl-C₁-C₆-alkyl, C₂-C₄-alkenylaminocarbonyl-C₁-C₆-alkyl, C₃-C₄-alkynylaminocarbonyl-C₁-C₆-alkyl, C₁-C₄-alkyl-C₃-C₄-alkenylaminocarbonyl-C₁-C₆-alkyl, C₁-C₄-alkyl-C₃-C₄-alkynylaminocarbonyl-C₁-C₆-alkyl, C₃-C₆-(α-alkylalkylidene)iminoxy-C₂-C₆-alkyl, cyclopropylmethyl, C₁-C₆-alkylamino, C₁-C₆-dialkylamino, C₁-C₆-alkylideneimino- or α-(C₁-C₄-alkyl)-C₂-C₆-alkylideneimino; and
is halogen, fluorine, chlorine, bromine or iodine.

2. A substituted trifluoromethylpyridine as claimed in claim 1 where
R¹ is amino or hydrazino,
R² is chlorine or fluorine,
R³ is C₁-C₄-alkyl, C₂-C₄-alkenyl or C₂-C₄-alkynyl, it being possible for these groups to have attached to them up to 6 halogen atoms, C₃-C₆-cycloalkyl which, in turn, can have attached to it up to 3 methyl radicals, C₁-C₂-alkoxy-C₂-C₄-alkoxycarbonyl-C₁-C₄-alkyl, C₁-C₄-cyanoalkyl, C₁-C₂-alkoxy-C₂-C₄-alkyl, 3-oxetanyl, carboxyl-C₁-C₄-alkyl, C₁-C₄-alkoxycarbonyl-C₁-C₄-alkyl, C₁-C₂-alkoxy-C₂-C₄-alkoxycarbonyl-C₁-C₄-alkyl, C₁-C₂-alkylaminocarbonyl-C₁-C₄-alkyl, C₃-C₄-(α-alkylidene)iminoxy-C₂-C₄-alkyl, cyclopropylmethyl, C₁-C₄-alkylamino, C₁-C₄-dialkylamino, C₁-C₄-alkylideneimino or α-(C₁-C₂-alkyl)-C₂-C₄-alkylideneimino.

3. A process for the preparation of trifluoromethylpyridines of the general formula I 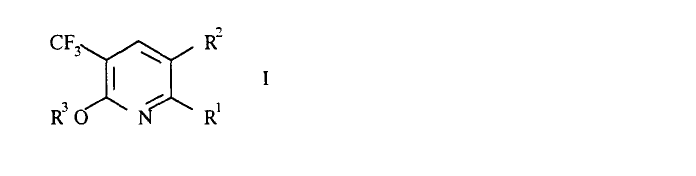 as claimed in claim 1, which comprises reacting a 3-halo-2,6-difluoro-5-trifluoromethylpyridine of the formula III 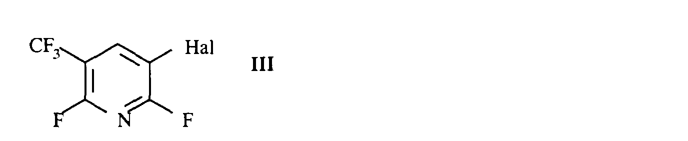 in succession with ammonia or hydrazine to give the 2-amino- or 2-hydrazino-3-halo-6-fluoro-5-trifluoromethylpyridine of the formula IV or IV' 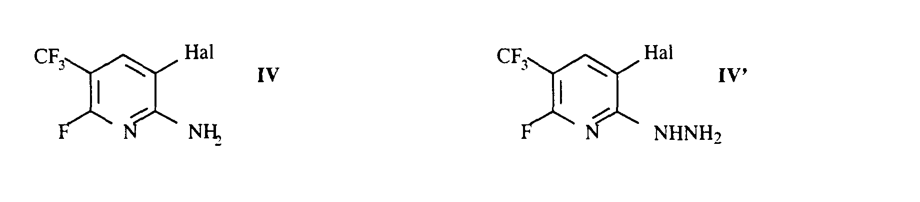 in the presence or absence of an organic auxiliary base and of a solvent, and then reacting the product with an alcohol of the general formula V
HOR³ V
or an alkali metal salt or alkaline earth metal salt thereof in the presence or absence of a base and of a solvent.

4. 2-Amino-3-chloro-6-methoxy-5-trifluoromethylpyridine.

5. 2-Amino-3-chloro-6-propargyloxy-5-trifluoromethylpyridine.

6. 2-Amino-3-chloro-6-methoxycarbonylmethoxy-5-trifluoromethylpyridine.

7. 2-Amino-3-chloro-6-ethoxy-5-trifluoromethylpyridine.

## Revendications

1. Trifluorométhylpyridines substituées de formule générale I 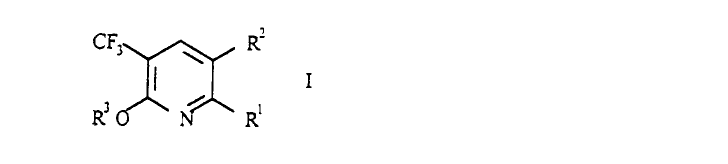 dans laquelle les symboles ont les significations suivantes :
R¹ : un groupe amino ou hydrazino ;
R² : un halogène ; et
R³ : un groupe alkyle en C1-C6, alcényle en C2-C6 ou alcynyle en C2-C6, ces groupes pouvant porter jusqu'à six atomes d'halogènes,
un groupe cycloalkyle en C3-C6 qui peut porter jusqu'à trois groupes alkyle en C1-C3, un groupe cyano-alkyle en C1-C6, (alcoxy en C1-C4)alkyle en C2-C6, 3-oxétanyle, carboxy-alkyle en C1-C6, (alcoxy en C1-C6)carbonyl-alkyle en C1-C6, (alcoxy en C1-C4)alcoxy en C2-C4-carbonyl-alkyle en C1-C6, aminocarbonyl-alkyle en C1-C6, (alkyle en C1-C4)aminocarbonyl-alkyle en C1-C6, di-(alkyle en C1-C4)aminocarbonyl-alkyle en C1-C6, (alcényle en C2-C4)aminocarbonyl-alkyle en C1-C6, (alcynyle en C3-C4)aminocarbonyl-alkyle en C1-C6, (alkyle en C1-C4)alcényle en C3-C4-aminocarbonyl-alkyle en C1-C6, (alkyle en C1-C4)alcynyle en C3-C4-aminocarbonyl-alkyle en C1-C6, (alphaalkylalkylidène en C3-C6)iminoxy-alkyle en C2-C6, cyclopropylméthyle, alkylamino en C1-C6, di-(alkyle en C1-C6)amino, alkylidène-imino en C1-C6 ou alpha-(alkyle en C1-C4)alkylidène en C2-C6-imino ; et
un halogène, le fluor, le chlore, le brome ou l'iode.

2. Trifluorométhylpyridines substituées selon la revendication 1 pour lesquelles
R¹ représente un groupe amino ou hydrazino,
R² représente le chlore ou le fluor,
R³ représente un groupe alkyle en C1-C4, alcényle en C2-C4 ou alcynyle en C2-C4, ces groupes pouvant porter jusqu'à six atomes d'halogènes,
un groupe cycloalkyle en C3-C6 qui peut lui-même porter jusqu'à trois groupes méthyle, un groupe (alcoxy en C1-C2)alcoxy en C2-C4-carbonyl-alkyle en C1-C4, un groupe cyano-alkyle en C1-C4, (alcoxy en C1-C2)alkyle en C2-C4, 3-oxétanyle, carboxylalkyle en C1-C4, (alcoxy en Cl-C4)carbonyl-alkyle en C1-C4, (alcoxy en Cl-C2)alcoxy en C2-C4-carbonyl-alkyle en C1-C4, (alkyle en Cl-C2)aminocarbonylalkyle en C1-C4, (alpha-alkylidène en C3-C4)iminoxy-alkyle en C2-C4, cyclopropylméthyle, alkylamino en C1-C4, di-(alkyle en C1-C4)amino, alkylidène en C1-C4-imino ou alpha-(alkyle en C1-C2)alkylidène en C2-C4-imino.

3. Procédé de préparation des trifluorométhylpyridines de formule générale I 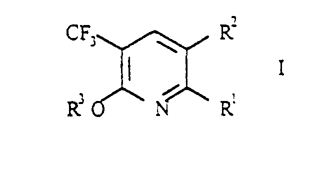 selon la revendication 1, **caractérisé par le fait que** l'on fait réagir une 3-halogéno-2,6-difluoro-5-trifluorométhyl-pyridine de formule III 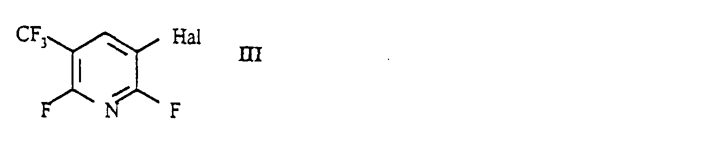 successivement avec l'ammoniac ou l'hydrazine, ce qui donne la 2-amino ou respectivement la 2-hydrazino-3-halogéno-6-fluoro-5-trifluorométhyl-pyridine de formule IV ou IV' 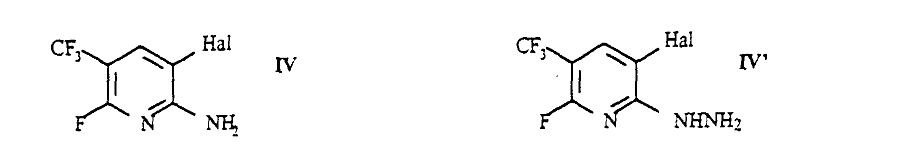 éventuellement en présence d'une base organique auxiliaire et d'un solvant, après quoi on fait réagir avec un alcool de formule générale V
HOR³ V
ou l'un de ses sels alcalins ou alcalino-terreux, éventuellement en présence d'une base ou d'un solvant.

4. La 2-amino-3-chloro-6-méthoxy-5-trifluorométhyl-pyridine.

5. La 2-amino-3-chloro-6-propargyloxy-5-trifluorométhyl-pyridine.

6. La 2-amino-3-chloro-6-méthoxycarbonyl-méthoxy-5-trifluorométhyl-pyridine.

7. La 2-amino-3-chloro-6-éthoxy-5-trifluorométhyl-pyridine.
